# EUROPEAN PATENT APPLICATION

(11) **EP 2 003 107 A1**
(43) Date of publication of application: **17.12.2008**
(21) Application number: 07740585.0
(22) Date of filing: 30.03.2007
(51) Int. Cl.: C07C 15/28, C09K 11/06, H01L 51/50

(54) **BISANTHRACENE DERIVATIVE AND ORGANIC ELECTROLUMINESCENT DEVICE USING THE SAME**

(30) Priority: 03.04.2006 JP 2006102335
(71) Applicant: Idemitsu Kosan Co., Ltd., Chiyoda-ku Tokyo 100-8321 (JP)
(72) Inventor: KUBOTA, Mineyuki, Sodegaura-shi Chiba 299-0293 (JP)
(74) Representative: Gille Hrabal Struck Neidlein Prop Roos
(86) International application number: PCT/JP2007/057149
(87) International publication number: WO 2007/116828

(57) **Abstract**

A bisanthracene derivative having a specific structure in which two anthracene groups are bonded via a bonding group composed of naphthylene group and p-phenylene group, and an organic electroluminescence device having an organic thin film layer, which has one layer or a plurality of layers including at least a light emitting layer, is disposed between a cathode and an anode and contains the bisanthracene derivatives singly or as a component of a mixture are provided. The electroluminescence device has a long life.

## Description

### TECHNICAL FIELD

The present invention relates to a bisanthracene derivative and an electroluminescence (referred to as EL, hereinafter) device using the same. More particularly, the present invention relates to an organic EL device having a long life and a bisanthracene derivative realizing the device.

### BACKGROUND ART

An organic EL device is a spontaneous light emitting device which utilizes the principle that a fluorescent substance emits light by energy of recombination of holes injected from an anode and electrons injected from a cathode when an electric field is applied. Since an organic EL device of the laminate type driven under a low electric voltage was reported by C. W. Tang of Eastman Kodak Company (C. W. Tang and S. A. Vanslyke, Applied Physics Letters, Volume 51, Pages 913, 1987), many studies have been conducted on organic EL devices using organic materials as the constituting materials. Tang et al. used tris(8-hydroxyquinolinolato)-aluminum for the light emitting layer and a triphenyldiamine derivative for the hole transporting layer. Advantages of the laminate structure are that the efficiency of hole injection into the light emitting layer can be increased, that the efficiency of forming excited particles which are formed by blocking and recombining electrons injected from the cathode can be increased, and that excited particles formed within the light emitting layer can be enclosed. As the structure of the organic EL device, a two-layered structure having a hole transporting (injecting) layer and an electron transporting and light emitting layer and a three-layered structure having a hole transporting (injecting) layer, a light emitting layer and an electron transporting (injecting) layer are well known. To increase the efficiency of recombination of injected holes and electrons in the devices of the laminate type, the structure of the device and the process for forming the device have been studied.

As the light emitting material, chelate complexes such as tris(8-quinolinolato)aluminum, coumarine derivatives, tetraphenylbutadiene derivatives, distyrylarylene derivatives and oxadiazole derivatives are known. It is reported that light in the visible region ranging from blue light to red light can be obtained by using these light emitting materials, and development of a device exhibiting color images is expected (for example, Patent Reference 1, Patent Reference 2 and Patent Reference 3).

Devices using a bisanthracene derivative as the light emitting material are disclosed in Patent References 4 to 7. The bisanthracene derivatives are used as the material emitting blue light. However, the life of the device is not sufficient, and improvement in the life of the device has been required.
[Patent Reference 1] Japanese Patent Application Laid-Open No. Heisei 8(1996)-239655
[Patent Reference 2] Japanese Patent Application Laid-Open No. Heisei 7(1995)-138561
[Patent Reference 3] Japanese Patent Application Laid-Open No. Heisei 3(1991)-200289
[Patent Reference 4] Japanese Patent Application Laid-Open No. Heisei 8(1996)-12600
[Patent Reference 5] Japanese Patent Application Laid-Open No. 2000-344691
[Patent Reference 6] Japanese Patent Application Laid-Open No. 2004-2351
[Patent Reference 7] Japanese Patent Application Laid-Open No. 2005-15420

### DISCLOSURE OF THE INVENTION

### Problems to be overcome by the Invention

The present invention has been made to overcome the above problem and has an object of providing an organic EL device having a long life and a novel bisanthracene derivative realizing the device.

### Means for overcoming the Problems

As the result of intensive studies by the present inventors to achieve the above object, it was found that an organic EL device having a long life could be obtained by using a bisanthracene derivative having a specific structure in which two anthracene groups are bonded via a bonding group composed of naphthylene group and p-phenylene group. The present invention has been completed based on the knowledge.

The present invention provides a bisanthracene derivative represented by following general formula (1): 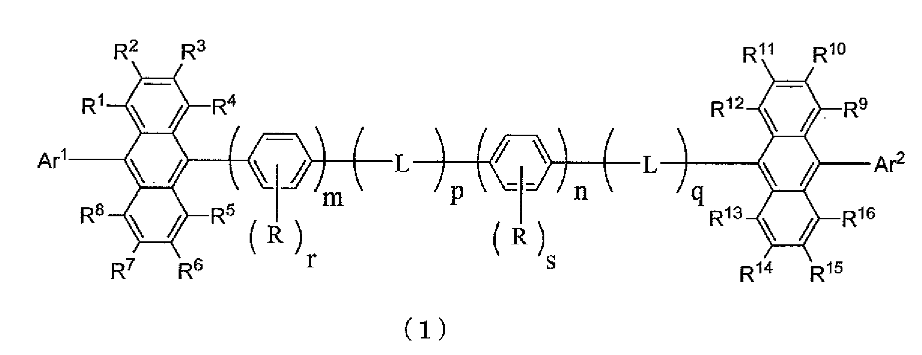 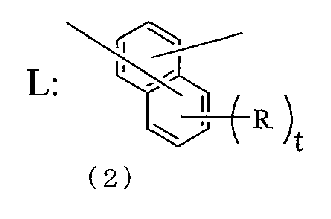 wherein
L represents a naphthylene group represented by above general formula (2);
Ar¹ and Ar² each independently represent a substituted or unsubstituted aromatic hydrocarbon group having 6 to 50 ring carbon atoms or a substituted or unsubstituted condensed aromatic hydrocarbon group having 10 to 50 ring carbon atoms;
R¹ to R¹⁶ each independently represent hydrogen atom, a substituted or unsubstituted aromatic group having 6 to 50 ring carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 ring carbon atoms, a substituted or unsubstituted alkoxyl group having 1 to 50 carbon atoms, a substituted or unsubstituted aralkyl group having 6 to 50 carbon atoms, a substituted or unsubstituted aryloxyl group having 5 to 50 ring carbon atoms, a substituted or unsubstituted arylthio group having 5 to 50 ring carbon atoms, a substituted or unsubstituted alkoxycarbonyl group having 1 to 50 carbon atoms, a substituted or unsubstituted silyl group, carboxyl group, a halogen atom, cyano group, nitro group or hydroxyl group;
R represents a substituted or unsubstituted aromatic group having 6 to 50 ring carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 ring carbon atoms, a substituted or unsubstituted alkoxyl group having 1 to 50 carbon atoms, a substituted or unsubstituted aralkyl group having 5 to 50 ring carbon atoms and 6 to 50 carbon atoms, a substituted or unsubstituted aryloxyl group having 5 to 50 ring carbon atoms, a substituted or unsubstituted arylthio group having 5 to 50 ring carbon atoms, a substituted or unsubstituted alkoxycarbonyl group having 1 to 50 carbon atoms, a substituted or unsubstituted silyl group, carboxyl group, a halogen atom, cyano group, nitro group or hydroxyl group;
m and n each represent an integer of 0 to 5, and m+n represents an integer of 1 to 5;
p and q each represent an integer of 0 to 5, and p+q represents an integer of 1 to 5;
r and s each represent an integer of 0 to 4;
t represents an integer of 0 to 6; and
when m, n, p, q, r, s and t represent integers of 1 or greater, atoms and groups represented by a plurality of L or R may be same with or different from each other.

The present invention also provides an organic electroluminescence device comprising a cathode, an anode and an organic thin film layer which comprises one layer or a plurality of layers comprising at least a light emitting layer and is disposed between the cathode and the anode, wherein the organic thin film layer comprises at least one compound selected from bisanthracene derivatives described above singly or as a component of a mixture.

### The effect of the Invention

The organic EL device comprising the bisanthracene derivative of the present invention has a long life.

### THE MOST PREFERRED EMBODIMENT TO CARRY OUT THE INVENTION

The bisanthracene derivative of the present invention is represented by the following general formula (1):

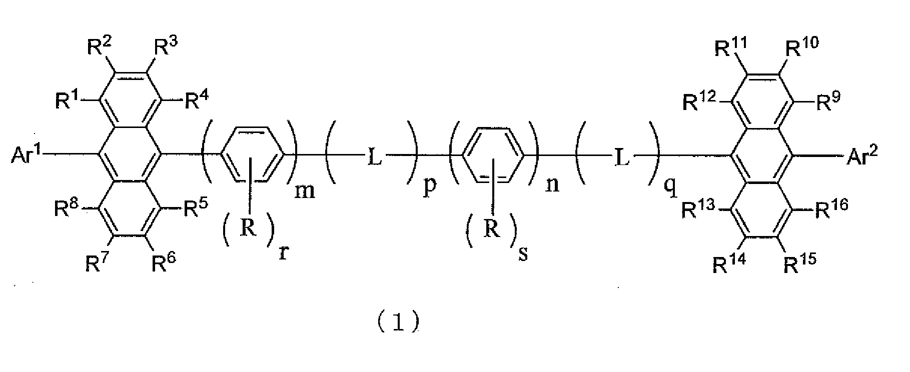

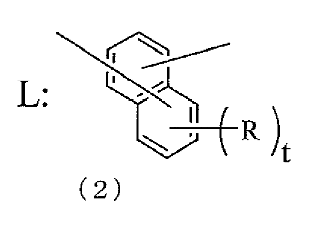

In general formula (1), L represents a naphthylene group represented by the above general formula (2) and preferably a naphthylene group represented by one of the following general formulae (3) to (5):

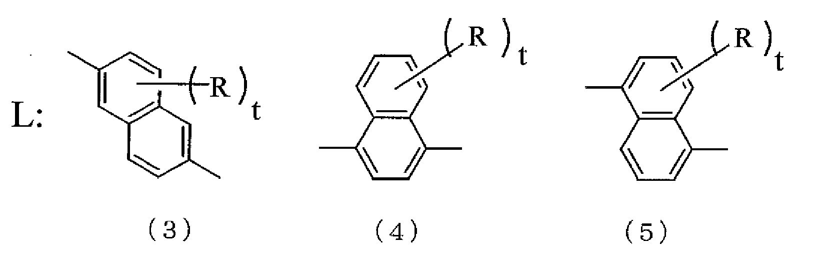

In general formula (1), Ar¹ and Ar² each independently represent a substituted or unsubstituted aromatic hydrocarbon group having 6 to 50 ring carbon atoms or a substituted or unsubstituted condensed aromatic hydrocarbon group having 10 to 50 ring carbon atoms.

Examples of the aromatic hydrocarbon group and the condensed aromatic hydrocarbon group represented by Ar¹ and Ar² include single ring aromatic hydrocarbon groups, multi-ring aromatic hydrocarbon groups, aromatic ring assembly groups and condensed multi-ring aromatic hydrocarbon groups. Specific examples include phenyl group, 1-naphthyl group, 2-naphthyl group, 1-anthryl group, 2-anthryl group, 9-anthryl group, 9-(10-phenyl)anthryl group, 9-(10-naphthyl-1-yl)anthryl group, 9-(10-naphthyl-2-yl)anthryl group, 1-phenanthryl group, 2-phenanthryl group, 3-phenanthryl group, 4-phenanthryl group, 9-phenanthryl group, 6-chrysenyl, group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, 3-methyl-2-naphthyl group, 4-methyl-1-naphthyl group and 4-methyl-1-anthryl group.

Among these groups, phenyl group, 1-naphthyl group, 2-naphthyl group, 9-(10-phenyl)anthryl group, 9-(10-naphthyl-1-yl)anthryl group, 9-(10-naphthyl-2-yl)anthryl group, 9-phenanthryl group, 1-pyrenyl group, 2-pyrenyl group, 4-pyrenyl group, 2-biphenylyl group, 3-biphenylyl group, 4-biphenylyl group, o-tolyl group, m-tolyl group, p-tolyl group and p-t-butylphenyl group are preferable, and phenyl group, 1-naphthyl group, 2-naphthyl group, 2-biphenylyl group, 3-biphenylyl group and 4-biphenylyl group are more preferable.

Examples of the substituent to the group represented by Ar¹ to Ar² include alkyl groups (such as methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloroisobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3-tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group, cyclopropyl group, cyclobutyl group, cyclopentyl group, cyclohexyl group, 4-methylcyclohexyl group, 1-adamantyl group, 2-adamantyl group, 1-norbornyl group and 2-norbornyl group), alkoxyl groups having 1 to 6 carbon atoms (such as ethoxyl group, methoxyl group, i-propoxyl group, n-propoxyl group, s-butoxyl group, t-butoxyl group, pentoxyl group, hexyloxyl group, cyclopentoxyl group and cyclohexyloxyl group), aryl groups having 5 to 40 nuclear atoms, amino groups substituted with aryl groups having 5 to 40 nuclear atoms, ester groups having aryl groups having 5 to 40 nuclear atoms, ester groups having alkyl groups having 1 to 6 carbon atoms, cyano group, nitro group and halogen atoms.

In general formula (1), R¹ to R¹⁶ each independently represent hydrogen atom, a substituted or unsubstituted aromatic hydrocarbon group having 6 to 50 ring carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 ring carbon atoms, a substituted or unsubstituted alkoxyl group having 1 to 50 carbon atoms, a substituted or unsubstituted aralkyl group having 5 to 50 ring carbon atoms and 6 to 50 ring carbon atoms, a substituted or unsubstituted aryloxyl group having 5 to 50 ring carbon atoms, a substituted or unsubstituted arylthio group having 5 to 50 ring carbon atoms, a substituted or unsubstituted alkoxycarbonyl group having 1 to 50 carbon atoms, a substituted or unsubstituted silyl group, carboxyl group, a halogen atom, cyano group, nitro group or hydroxyl group.

In general formula (1), R represents hydrogen atom, a substituted or unsubstituted aromatic hydrocarbon group having 6 to 50 ring carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 ring carbon atoms, a substituted or unsubstituted alkoxyl group having 1 to 50 carbon atoms, a substituted or unsubstituted aralkyl group having 5 to 50 ring carbon atoms and 6 to 50 carbon atoms, a substituted or unsubstituted aryloxyl group having 5 to 50 ring carbon atoms, a substituted or unsubstituted arylthio group having 5 to 50 ring carbon atoms, a substituted or unsubstituted alkoxycarbonyl group having 1 to 50 carbon atoms, a substituted or unsubstituted silyl group, carboxyl group, a halogen atom, cyano group, nitro group or hydroxyl group.

Examples of the aromatic hydrocarbon group represented by R and R¹ to R¹⁶ include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)-phenyl group, 3-methyl-2-naphthyl group, 4-methyl-1-naphthyl group, 4-methyl-1-anthryl group, 4'-methylbiphenylyl group and 4"-t-butyl-p-terphenyl-4-yl group.

Examples of the aromatic heterocyclic group represented by R and R¹ to R¹⁶ include 1-pyrrolyl group, 2-pyrrolyl group, 3-pyrrolyl group, pyradinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 9-carbazolyl group, 1-phenanthridinyl group, 2-phenanthr-idinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenauthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 10-phenothiazinyl group, 1-phenoxazinyl group, 2-phenoxazinyl group, 3-phenoxazinyl group, 4-phenoxazinyl group, 10-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group and 4-t-butyl-3-indolyl group.

Examples of the alkyl group represented by R and R¹ to R¹⁶ include methyl group, ethyl group, propyl group, isopropyl group, n-butyl group, s-butyl group, isobutyl group, t-butyl group, n-pentyl group, n-hexyl group, n-heptyl group, n-octyl group, hydroxymethyl group, 1-hydroxyethyl group, 2-hydroxyethyl group, 2-hydroxyisobutyl group, 1,2-dihydroxyethyl group, 1,3-dihydroxyisopropyl group, 2,3-dihydroxy-t-butyl group, 1,2,3-trihydroxypropyl group, chloromethyl group, 1-chloroethyl group, 2-chloroethyl group, 2-chloro- isobutyl group, 1,2-dichloroethyl group, 1,3-dichloroisopropyl group, 2,3-dichloro-t-butyl group, 1,2,3-trichloropropyl group, bromomethyl group, 1-bromoethyl group, 2-bromoethyl group, 2-bromoisobutyl group, 1,2-dibromoethyl group, 1,3-dibromoisopropyl group, 2,3-dibromo-t-butyl group, 1,2,3-tribromopropyl group, iodomethyl group, 1-iodoethyl group, 2-iodoethyl group, 2-iodoisobutyl group, 1,2-diiodoethyl group, 1,3-diiodoisopropyl group, 2,3-diiodo-t-butyl group, 1,2,3-triiodopropyl group, aminomethyl group, 1-aminoethyl group, 2-aminoethyl group, 2-aminoisobutyl group, 1,2-diaminoethyl group, 1,3-diaminoisopropyl group, 2,3-diamino-t-butyl group, 1,2,3-triaminopropyl group, cyanomethyl group, 1-cyanoethyl group, 2-cyanoethyl group, 2-cyanoisobutyl group, 1,2-dicyanoethyl group, 1,3-dicyanoisopropyl group, 2,3-dicyano-t-butyl group, 1,2,3- tricyanopropyl group, nitromethyl group, 1-nitroethyl group, 2-nitroethyl group, 2-nitroisobutyl group, 1,2-dinitroethyl group, 1,3-dinitroisopropyl group, 2,3-dinitro-t-butyl group, 1,2,3-trinitropropyl group.

Examples of the cycloalkyl group represented by R and R¹ to R¹⁶ include cyclopropyl group, cyclobutyl group, cyclopentyl group, cyclohexyl group, 4-methylcyclohexyl group, 1-adamantyl group, 2-adamantyl group, 1-norbornyl group and 2-norbornyl group.

The alkoxyl group represented by R and R¹ to R¹⁶ is a group represented by -OY. Examples of the group represented by Y include the groups described as the examples of the alkyl group.

Examples of the aralkyl group represented by R and R¹ to R¹⁶ include benzyl group, 1-phenylethyl group, 2-phenylethyl group, 1-phenylisopropyl group, 2-phenylisopropyl group, phenyl-t-butyl group, α-naphthylmethyl group, 1-α-naphthylethyl group, 2-α-naphthylethyl group, 1-α-naphthylisopropyl group, 2-α-naphthylisopropyl group, β-naphthylmethyl group, 1-β-naphthylethyl group, 2-β-naphthylethyl group, 1-β-naphthylisopropyl group, 2-β-naphthylisopropyl group, 1-pyrrolylmethyl group, 2-(1-pyrrolyl)ethyl group, p-methylbenzyl group, m-methylbenzyl group, o-methylbenzyl group, p-chlorobenzyl group, m-chlorobenzyl group, o-chlorobenzyl group, p-bromobenzyl group, m-bromobenzyl group, o-bromobenzyl group, p-iodobenzyl group, m-iodobenzyl group, o-iodobenzyl group, p-hydroxybenzyl group, m-hydroxybenzyl group, o-hydroxybenzyl group, p-aminobenzyl group, m-aminobenzyl group, o-aminobenzyl group, p-nitrobenzyl group, m-nitrobenzyl group, o-nitrobenzyl group, p-cyanobenzyl group, m-cyanobenzyl group, o-cyanobenzyl group, 1-hydroxy-2-phenylisopropyl group and 1-chloro-2-phenylisopropyl group.

The aryloxyl group represented by R and R¹ to R¹⁶ is a group represented by -OY'. Examples of the group represented by Y' include phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-mothyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyradinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxanyl group, 5-quinoxanyl group, 6-quinoxanyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,8-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiazinyl group, 2-phenothiazinyl group, 3-phenothiazinyl group, 4-phenothiazinyl group, 1-phenoxazinyl group, 2-phenoxazinyl group, 3-phenoxazinyl group, 4-phenoxazinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butylpyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl- 3-indolyl group and 4-t-butyl-3-indolyl group.

The arylthio group represented by R and R¹ to R¹⁶ is represented by -SY'. Examples of the group represented by Y' include the groups described above as the examples of the group represented by Y' in the aryloxyl group.

The alkoxycarbonyl group represented by R and R¹ to R¹⁶ is represented by -COOZ. Examples of the group represented by Z include the groups described above as the examples of the alkyl group.

Examples of the silyl group represented by R and R¹ to R¹⁶ include trimethylsilyl group, triethylsilyl group, t-butyldimethylsilyl group, vinyldimethylsilyl group and propyldimethyl- silyl group.

Examples of the halogen atom represented by R and R¹ to R¹⁶ include fluorine atom, chlorine atom, bromine atom and iodine atom.

Examples of the substituent to the group represented by R and R¹ to R¹⁶ include the substituents described as the examples of the substituent to the group represented by Ar¹ and Ar².

In general formula (1), m and n each represent an integer of 0 to 5, preferably 0 to 3 and more preferably 0 to 2, and m+n represents an integer of 1 to 5 and preferably 1 to 3.

In general formula (1), p and q each represent an integer of 0 to 5, preferably 0 to 3 and more preferably 0 to 2, and p+q represents an integer of 1 to 5 and preferably 1 to 3.

In general formula (1), r and s each represent an integer of 0 to 4 and preferably 0 to 2, and t represents an integer of 0 to 6 and preferably 0 to 2.

When m, n, p, q, r, s and t represent integers of 1 or greater, atoms and groups represented by a plurality of L or R may be same with or different from each other.

Specific examples of the bisanthracene derivatives represented by general formula (1) of the present invention are shown in the following. However, the bisanthracene derivative of the present invention is not limited to these compounds.

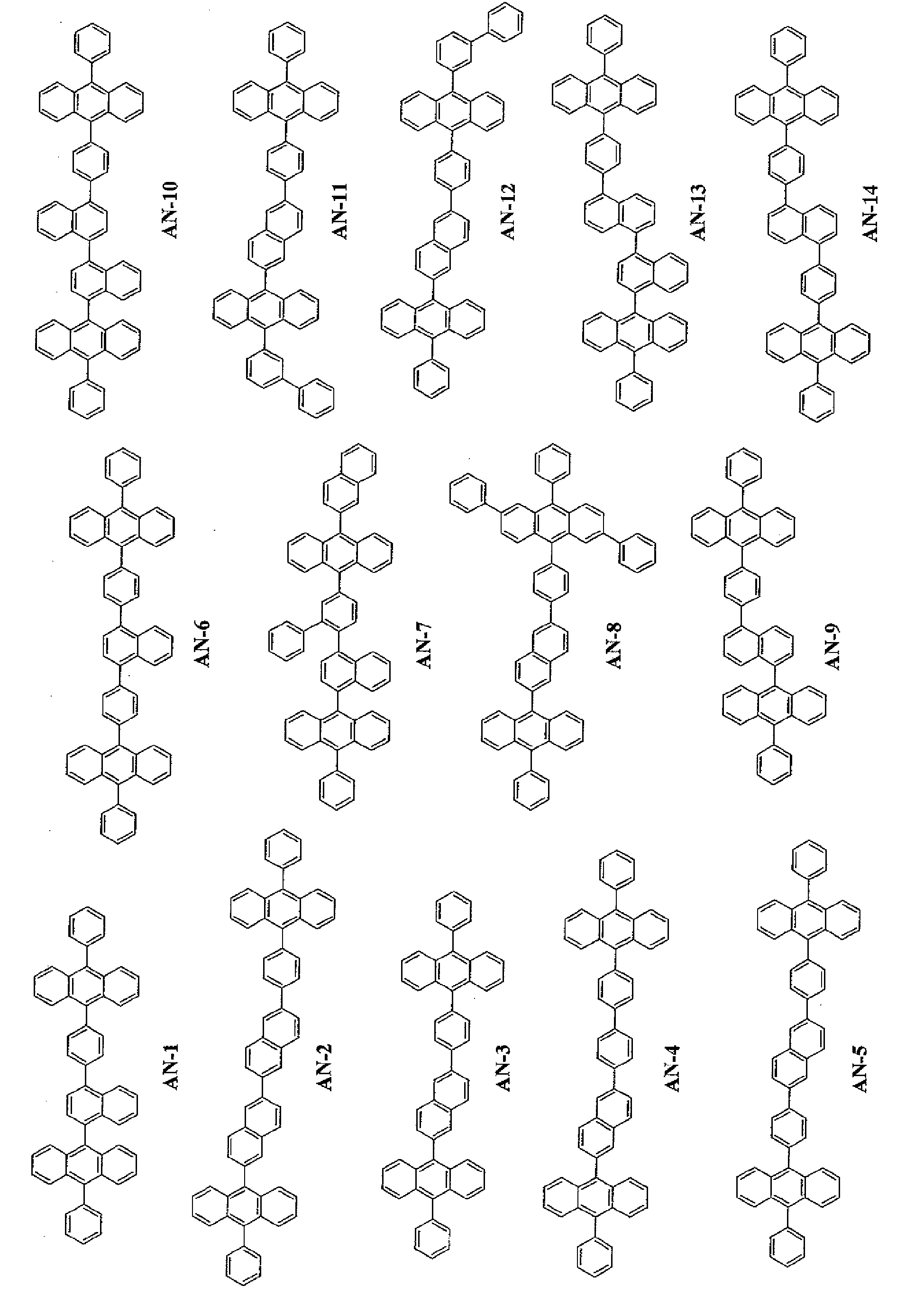

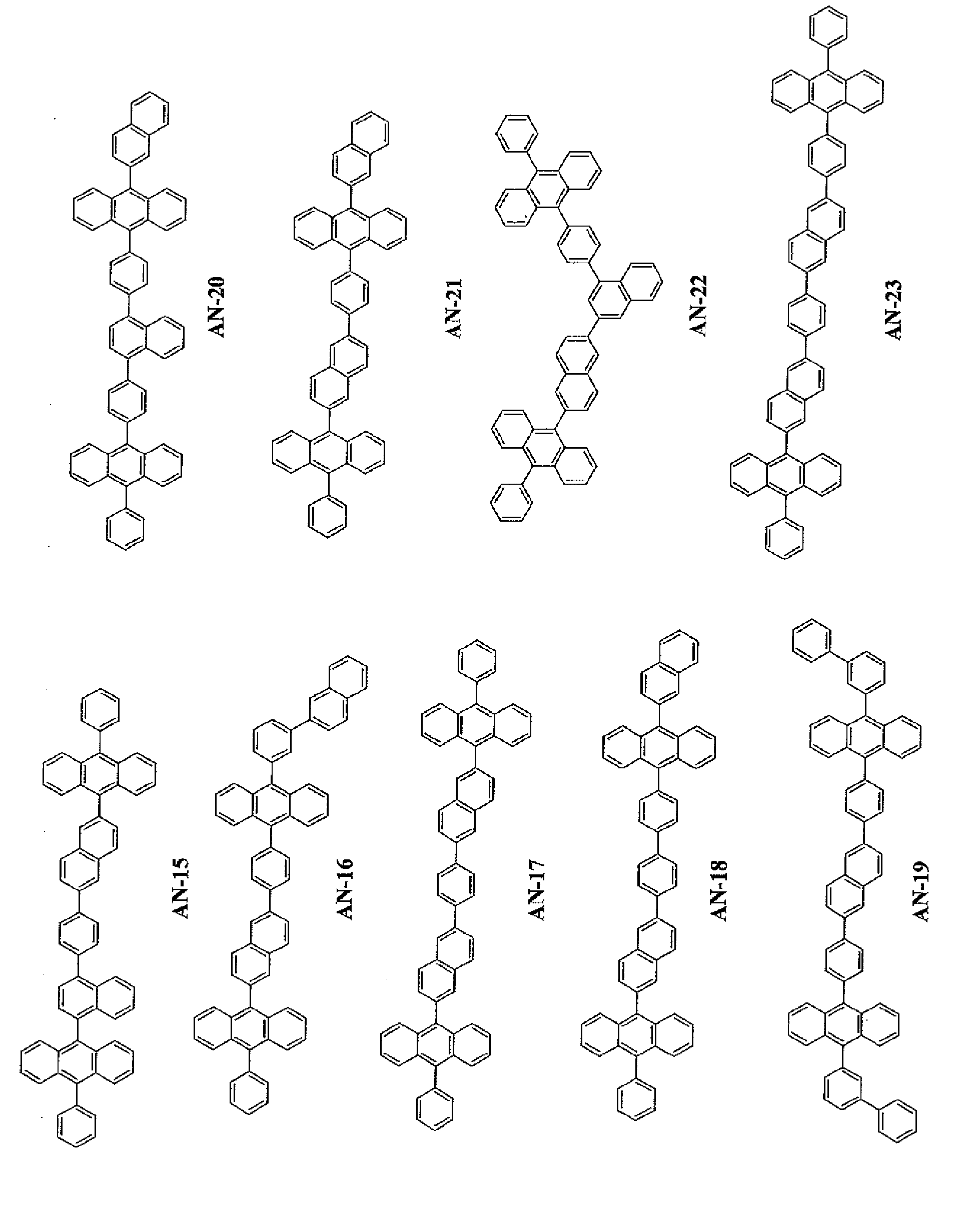

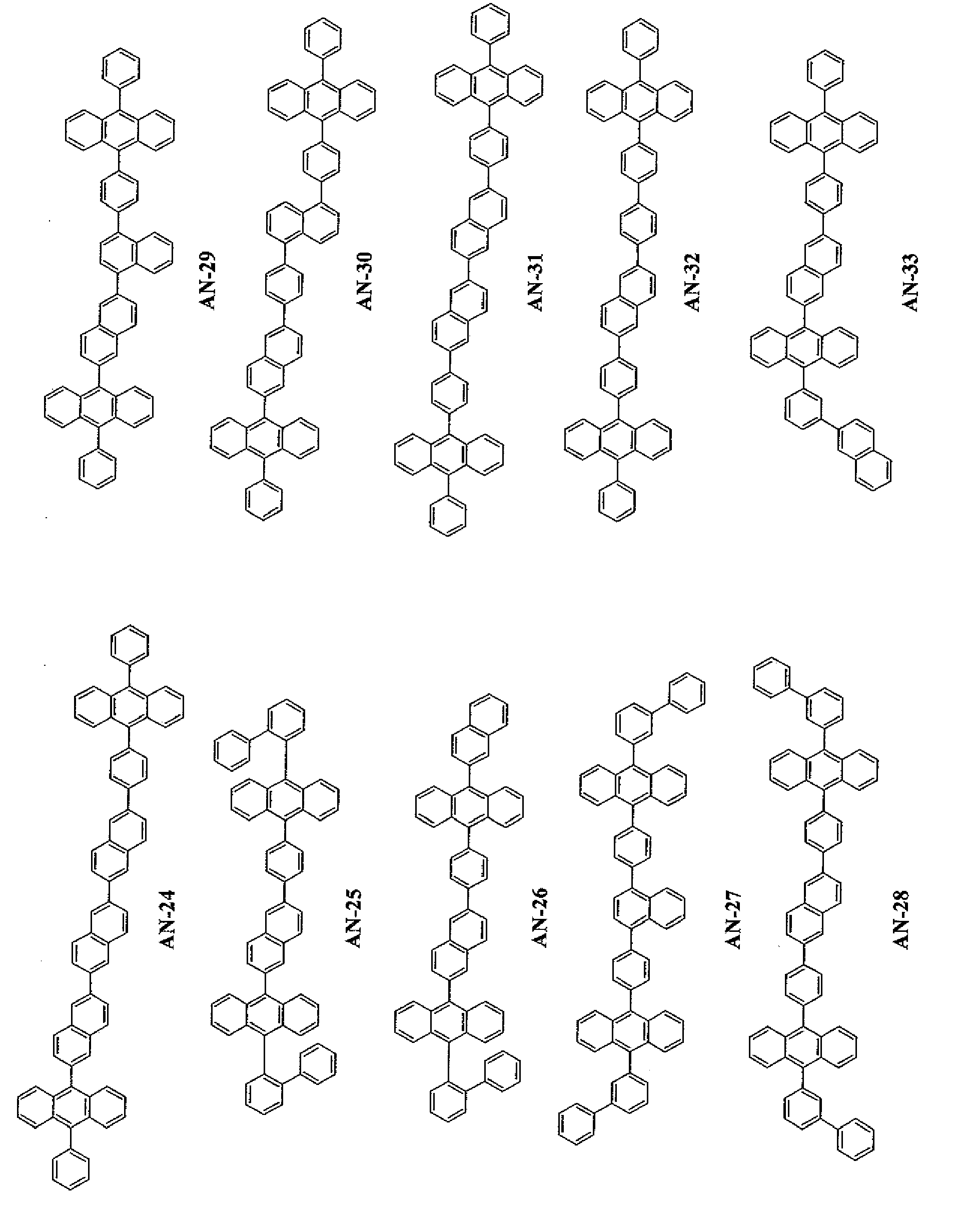

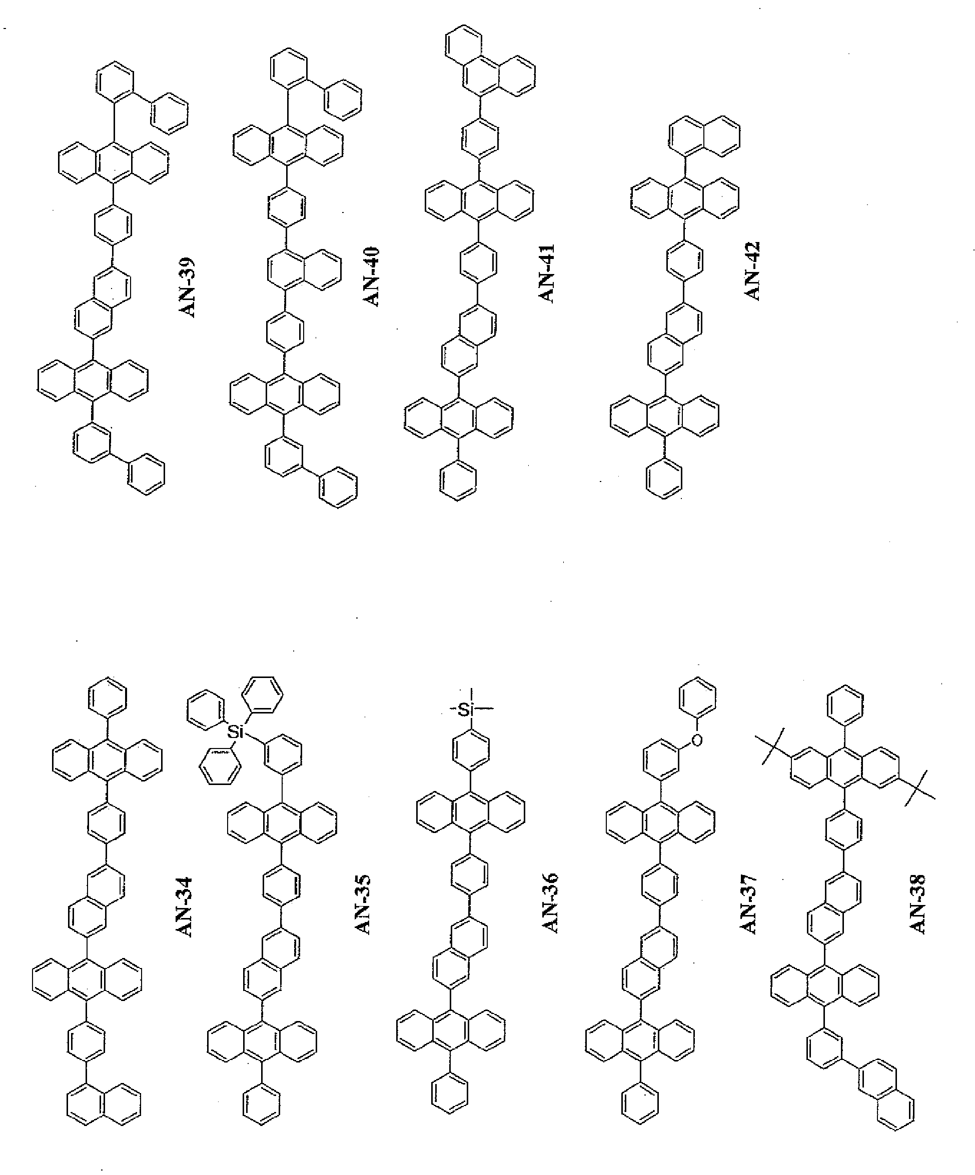

The bisanthracene derivative of the present invention can be synthesized in accordance with the Suzuki coupling reaction using an arylboronic acid derivative and a halogenated aryl derivative, which are synthesized in accordance with conventional processes. Examples of these compounds are shown as 1 to 11 in the following Table.

In the Table, L, Ar¹, Ar², R¹ to R¹⁶, R, m, n, p, q, r and s are as defined above, R¹⁷ to R²⁰ each independently represent hydroxyl group or an alkoxyl group, and X¹ and X² each represent a halogen atom.

| | Arylboronic Acid Derivative | Halogenated Aryl Derivative |
|---|---|---|
| 1 | 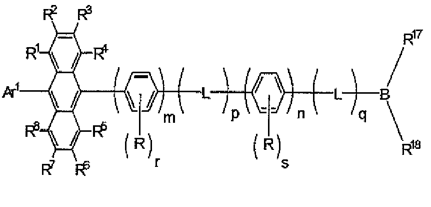 | 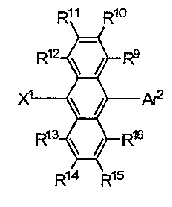 |
| 2 | 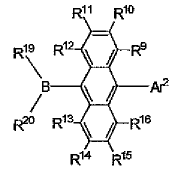 | 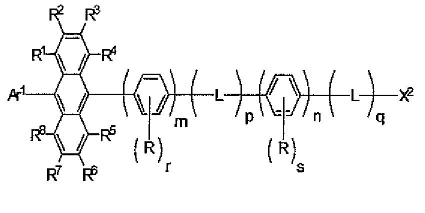 |
| 3 | 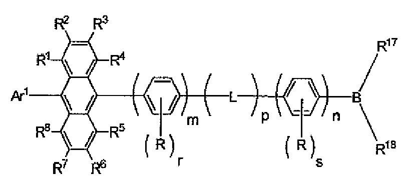 | 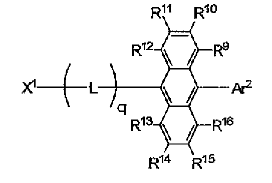 |
| 4 | 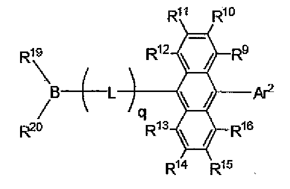 | 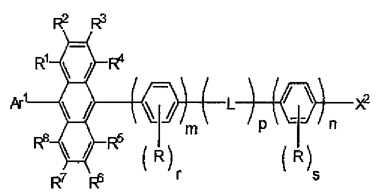 |
| 5 | 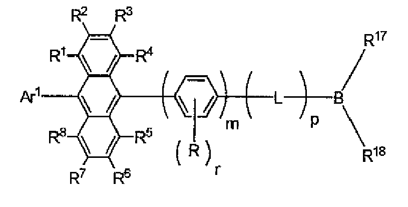 | 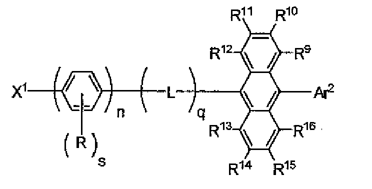 |
| 6 | 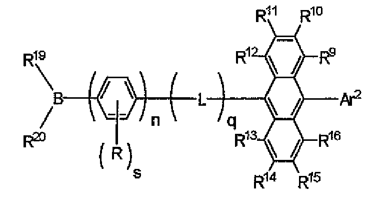 | 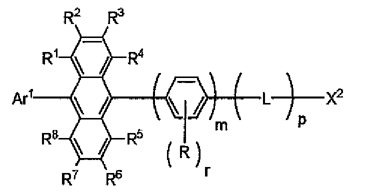 |
| 7 | 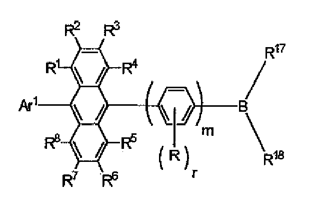 | 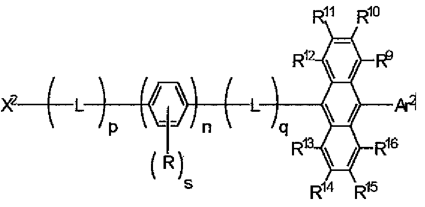 |
| 8 9 | 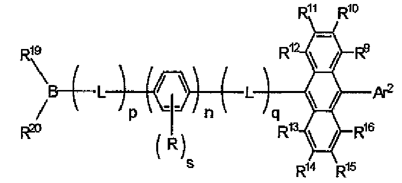 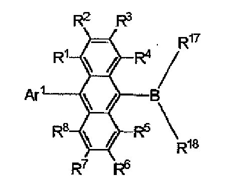 | 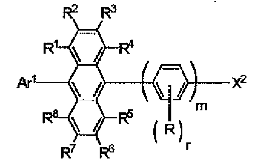 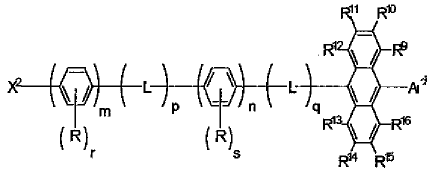 |
| 10 11 | 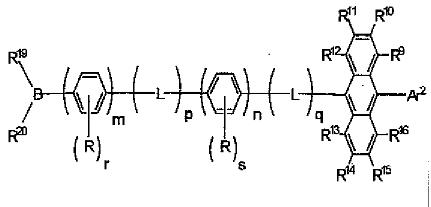 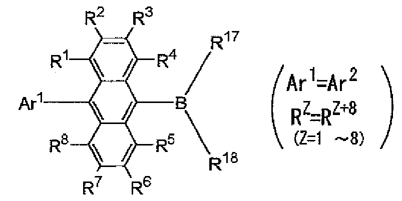 | 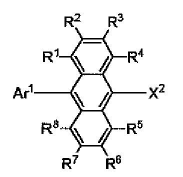 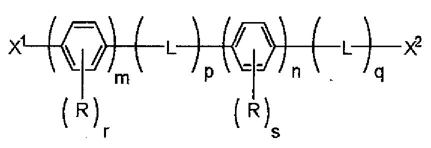 |

It is preferable that the bisanthracene derivative of the present invention is used as the light emitting material for organic EL devices and more preferably as the host material for organic EL devices.

The organic EL device of the present invention comprises a cathode, an anode and an organic thin film layer which comprises one layer or a plurality of layers comprising at least a light emitting layer and is disposed between the cathode and the anode, wherein the organic thin film layer comprises at least one compound selected from bisanthracene derivatives represented by general formula (1) shown above singly or as a component of a mixture.

In the organic EL device of the present invention, it is preferable that the light emitting layer further comprises an arylamine compound and/or a styrylamine compound.

As the styrylamine compound, compounds represented by the following general formula (A) are preferable: 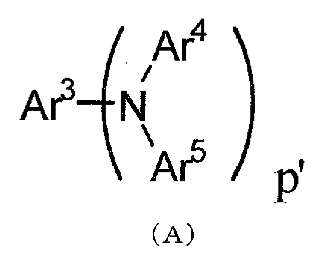 wherein
Ar³ represents a group selected from phenyl group, biphenyl group, terphenyl group, stilbene group and distyrylaryl groups, Ar⁴ and Ar⁵ each represent hydrogen atom or an aromatic hydrocarbon group having 6 to 20 carbon atoms, the groups represented by Ar³, Ar⁴ and Ar⁵ may be substituted, p' represents an integer of 1 to 4, and it is preferable that at least one of the groups represented by Ar⁴ and Ar⁵ is substituted with styryl group; and
at least one of the groups represented by Ar³ to Ar⁵ has a substituted or unsubstituted styryl group.

Examples of the aromatic hydrocarbon group having 6 to 20 carbon atoms include phenyl group, naphthyl group, anthranyl group, phenanthryl group and terphenyl group.

As the arylamine compound, compounds represented by the following general formula (B) are preferable 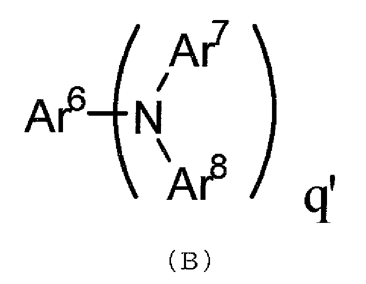 wherein Ar⁶ to Ar⁸ each represent a substituted or unsubstituted aryl group having 5 to 40 ring carbon atoms, and q' represents an integer of 1 to 4.

Examples of the aryl group having 5 to 40 ring carbon atoms include phenyl group, naphthyl group, anthranyl group, phenanthryl group, pyrenyl group, coronyl group, biphenyl group, terphenyl group, pyrrolyl group, furanyl group, thiophenyl group, benzothiophenyl group, oxadiazolyl group, diphenylanthranyl group, indolyl group, carbazolyl group, pyridyl group, benzoquinolyl group, fluoranthenyl group, acenaphthofluoranthenyl group, stilbene group, perylenyl group, chrysenyl group, pycenyl group, triphenylenyl group, rubicenyl group, benzoanthracenyl group, phenylanthranyl group, bisanthracenyl group and aryl groups represented by the following general formula (C) or expressed by the following formula (D). Among these groups, naphthyl group, anthranyl group, chrysenyl group, pyrenyl group and the aryl group expressed by formula (D) are preferable. 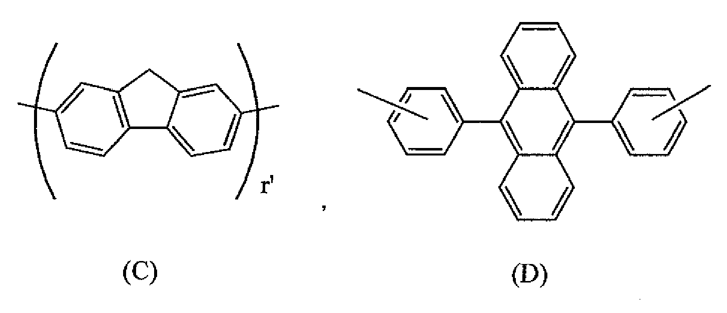 In general formula (C), r' represents an integer of 1 to 3.

Preferable examples of the substituent to the aryl group include alkyl groups having 1 to 6 carbon atoms such as ethyl group, methyl group, i-propyl group, n-propyl group, s-butyl group, t-butyl group, pentyl group, hexyl group, cyclopentyl group and cyclohexyl group; alkoxyl groups having 1 to 6 carbon atoms such as ethoxyl group, methoxyl group, i-propoxyl group, n-propoxyl group, s-butoxyl group, t-butoxyl group, pentoxyl group, hexyloxyl group, cyclopentoxyl group and cyclohexyloxyl group; aryl groups having 5 to 40 ring carbon atoms; amino groups substituted with an aryl group having 5 to 40 ring carbon atoms; ester groups having an aryl group having 5 to 40 ring carbon atoms; ester groups having an alkyl group having 1 to 6 carbon atoms; cyano group; nitro group; and halogen atoms.

The construction of the organic EL device of the present invention will be described in the following.

Typical examples of the construction of the organic EL device include :
(1) An anode / a light emitting layer / a cathode;
(2) An anode / a hole injecting layer / a light emitting layer / a cathode;
(3) An anode / a light emitting layer / an electron injecting layer / a cathode;
(4) An anode / a hole injecting layer / a light emitting layer / an electron injecting layer / a cathode;
(5) An anode / an organic semiconductor layer / a light emitting layer / a cathode;
(6) An anode / an organic semiconductor layer / an electron barrier layer / a light emitting layer / a cathode;
(7) An anode / an organic semiconductor layer / a light emitting layer / an adhesion improving layer / a cathode;
(8) An anode / a hole injecting layer / a hole transporting layer / a light emitting layer / an electron injecting layer / a cathode;
(9) An anode / an insulating layer / a light emitting layer / an insulating layer / a cathode;
(10) An anode / an inorganic semiconductor layer / an insulating layer / a light emitting layer / an insulating layer / a cathode;
(11) An anode / an organic semiconductor layer / an insulating layer / a light emitting layer / an insulating layer / a cathode;
(12) An anode / an insulating layer / a hole injecting layer / a hole transporting layer / a light emitting layer / an insulating layer / a cathode; and
(13) An anode / an insulating layer / a hole injecting layer / a hole transporting layer / a light emitting layer / an electron injecting layer / a cathode.

Among the above constructions, construction (8) is preferable. However, the construction of the organic EL device is not limited to those shown above as the examples.

In the organic EL device of the present invention, it is preferable that the light emitting zone or the hole transporting zone comprises the bisanthracene derivative of the present invention among the constituting elements of the device although any of the organic layers may comprise the bisanthracene derivative. The content of the bisanthracene derivative is selected in the range of 30 to 100% by mole.

The organic EL device is, in general, prepared on a substrate transmitting light. The substrate transmitting light is the substrate supporting the organic EL device. It is preferable that the substrate transmitting light has a transmittance of light of 50% or greater in the visible region of 400 to 700 nm. It is also preferable that a flat and smooth substrate is used.

As the substrate transmitting light, for example, glass plates and synthetic resin plates are advantageously used. Examples of the glass plate include plates made of soda ash glass, glass containing barium and strontium, lead glass, aluminosilicate glass, borosilicate glass, barium borosilicate glass and quartz. Examples of the synthetic resin plate include plates made of polycarbonate resins, acrylic resins, polyethylene terephthalate resins, polyether sulfide resins and polysulfone resins.

The anode has the function of injecting holes into the hole transporting layer or the light emitting layer. It is effective that the anode has a work function of 4.5 eV or greater. Examples of the material for the anode used in the present invention include indium tin oxide alloys (ITO), indium zinc oxide alloys (IZO), tin oxide (NESA), gold, silver, platinum and copper. For the cathode, materials having a small work function are preferable for the purpose of injecting electrons into the electron transporting layer or the light emitting layer.

The anode can be prepared by forming a thin film of the electrode substance described above in accordance with a process such as the vapor deposition process and the sputtering process.

When the light emitted from the light emitting layer is obtained through the anode, it is preferable that the anode has a transmittance of the emitted light greater than 10%. It is also preferable that the sheet resistivity of the anode is several hundred Ω/□ or smaller. The thickness of the anode is, in general, selected in the range of 10 nm to 1 µm and preferably in the range of 10 to 200 nm although the range may be different depending on the used material.

The light emitting layer in the organic EL device of the present invention has the following functions:
(i) The injecting function: the function of injecting holes from the anode or the hole injecting layer and injecting electrons from the cathode or the electron injecting layer when an electric field is applied;
(ii) The transporting function: the function of transporting injected charges (electrons and holes) by the force of the electric field; and
(iii) The light emitting function: the function of providing the field for recombination of electrons and holes and leading the recombination to the emission of light.

As the process for forming the light emitting layer, a conventional process such as the vapor deposition process, the spin coating process and the LB process can be used. It is particularly preferable that the light emitting layer is a molecular deposit film. The molecular deposit film is a thin film formed by deposition of a material compound in the gas phase or a thin film formed by solidification of a material compound in a solution or in the liquid phase. In general, the molecular deposit film can be distinguished from the thin film formed in accordance with the LB process (the molecular accumulation film) based on the differences in aggregation structures and higher order structures and the functional differences caused by these structural differences.

As disclosed in Japanese Patent Application Laid-Open No. Showa 57(1982)-51781, the light emitting layer can also be formed by dissolving a binder such as a resin and the material compounds into a solvent to prepare a solution, followed by forming a thin film from the prepared solution in accordance with the spin coating process or the like.

In the present invention, where desired, the light emitting layer may comprise conventional light emitting materials other than the light emitting material comprising the bisanthracene derivative of the present invention, or a light emitting layer comprising other conventional light emitting material may be laminated to the light emitting layer comprising the light emitting material of the present invention as long as the object of the present invention is not adversely affected.

The hole injecting and transporting layer is a layer which helps injection of holes into the light emitting layer and transports the holes to the light emitting region. The layer exhibits a great mobility of holes and, in general, has an ionization energy as small as 5.5 eV or smaller. For the hole injecting and transporting layer, a material which transports holes to the light emitting layer under an electric field of a smaller strength is preferable. A material which exhibits, for example, a mobility of holes of at least 10⁻⁴ cm²/T-sec under application of an electric field of 10⁴ to 10⁶ V/cm is preferable. As the above material, a material can be selected as desired from materials which are conventionally used as the charge transporting material of holes in photoconductive materials and conventional materials which are used for the hole injecting layer in organic EL devices.

Examples include triazole derivatives (United States Patent No. 3,112,197), oxadiazole derivatives (United States Patent No. 3,189,447), imidazole derivatives (Japanese Patent Application Publication No. Showa 37(1962)-16096), polyarylalkane derivatives (United States Patent Nos. 3,615,402, 3,820,989 and 3,542,544; Japanese Patent Application Publication Nos. Showa 45(1970)-555 and Showa 51 (1976)-10983; and Japanese Patent Application Laid-Open Nos. Showa 51(1976)-93224, Showa 55(1980)-17105, Showa 56(1981)-4148, Showa 55(1980)-108667, Showa 55(1980)-156953 and Showa 56(1981)-36656)); pyrazoline derivatives and pyrazolone derivatives (United States Patent Nos. 3,180,729 and 4,278,746; and Japanese Patent Application Laid-Open Nos. Showa 55(1980)-88064, Showa 55(1980)-88065, Showa 49(1974)-105537, Showa 55(1980)-51086, Showa 56(1981)-80051, Showa 56(1981)-88141, Showa 57(1982)-45545, Showa 54(1979)-112637 and Showa 55(1980)-74546); phenylenediamine derivatives (United Sates Patent No. 3,615,404; Japanese Patent Application Publication Nos. Showa 51(1976)-10105, Showa 46(1971)-3712 and Showa 47(1972)-25336; and Japanese Patent Application Laid-Open Nos. Showa 54(1979)-53435, Showa 54(1979)-110536 and Showa 54(1979)-119925); arylamine derivatives (United States Patent Nos. 3,567,450, 3,180,703, 3,240,597, 3,658,520, 4,232,103, 4,175,961 and 4,012,376; Japanese Patent Application Publication Nos. Showa 49(1974)-35702 and Showa 39(1964)-27577; Japanese Patent Application Laid-Open Nos. Showa 55(1980)-144250, Showa 56(1981)-119132 and Showa 56(1981)-22437; and West German Patent No. 1,110,518); chalcone derivatives substituted with amino group (United States Patent No. 3,526,501); oxazole derivatives (United States Patent No. 3,257,203); styrylanthracene derivatives (Japanese Patent Application Laid-Open Nos. Showa 56(1981)-46234); fluorenone derivatives (Japanese Patent Application Laid-Open Nos. Showa 54(1979)-110837); hydrazone derivatives (United States Patent No. 3,717,462; and Japanese Patent Application Laid-Open Nos. Showa 54(1979)-59143, Showa 55(1980)-52063, Showa 55(1980)-52064, Showa 55(1980)-46760, Showa 55(1980)-85495, Showa 57(1982)-11350, Showa 57(1982)-148749 and Heisei 2(1990)-311591); stilbene derivatives (Japanese Patent Application Laid-Open Nos. Showa 61(1986)-210363, Showa 61(1986)-228451, Showa 61(1986)-14642, Showa 61,(1986)-72255, Showa 62(1987)-47646, Showa 62(1987)-36674, Showa 62(1987)-10652, Showa 62(1987)-30255, Showa 60(1985)-93455, Showa 60(1985)-94462, Showa 60(1985)-174749 and Showa 60(1985)-175052); silazane derivatives (United States Patent No. 4,950,950); polysilane-based compounds (Japanese Patent Application Laid-Open No. Heisei 2(1990)-204996); aniline-based copolymers (Japanese Patent Application Laid-Open No. Heisei 2(1990)-282263); and electrically conductive macromolecular oligomers (in particular, thiophene oligomers) disclosed in Japanese Patent Application Laid-Open No. Heisei 1(1989)-211399.

Besides the above materials which can be used as the material for the hole injecting layer, porphyrin compounds (compounds disclosed in Japanese Patent Application Laid-Open No. Showa 63(1988)-2956965); and aromatic tertiary amine compounds and styrylamine compounds (United States Patent No. 4,127,412 and Japanese Patent Application Laid-Open Nos. Showa 53(1978)-27033, Showa 54(1979)-58445, Showa 54(1979)-149634, Showa 54(1979)-64299, Showa 55(1980)-79450. Showa 55(1980)-144250, Showa 56(1981)-119132, Showa 61(1986)-295558, Showa 61(1986)-98353 and Showa 63(1988)-295695) are preferable, and the aromatic tertiary amines are more preferable.

Further examples include compounds having two condensed aromatic rings in the molecule which are described in the United States Patent No. 5,061,569 such as 4,4'-bis(N-(1-naphthyl)-N-phenylamino)-biphenyl (referred to as NPD, hereinafter) and a compound in which three triphenylamine units are bonded together in a star-burst shape, which is described in Japanese Patent Application Laid-Open No. Heisei 4(1992)-308688, such as 4,4',4"-tris(N-(3-methylphenyl)-N-phenylamino)-triphenylamine (referred to as MTDATA, hereinafter).

Besides the above bisanthracene derivatives, inorganic compounds such as Si of the p-type and SiC of the p-type can also be used as the material for the hole injecting layer.

The hole injecting and transporting layer can be formed by preparing a thin film of the above compound in accordance with a conventional process such as the vacuum vapor deposition process, the spin coating process, the casting process and the LB process. The thickness of the hole injecting and transporting layer is not particularly limited. In general, the thickness is 5 nm to 5 µm.

The organic semiconductor layer is a layer helping injection of holes or electrons into the light emitting layer. As the organic semiconductor layer, a layer having a conductivity of 10⁻¹⁰ S/cm or greater is preferable. As the material for the organic semiconductor layer, oligomers containing thiophene can be used, and conductive oligomers such as oligomers containing allylamine and conductive dendrimers such as dendrimers containing allylamine, which are disclosed in Japanese Patent Application Laid-Open No. Heisei 8(1996)-193191, can also be used.

The electron injecting and transporting layer is a layer which helps injection of electrons into the light emitting layer and transportation of the electrons to the light emitting region and exhibits a great mobility of electrons. The adhesion improving layer is an electron injecting layer comprising a material exhibiting improved adhesion with the cathode.

It is known that, in an organic EL device, emitted light is reflected at an electrode (the cathode in the present case), and the light emitted and obtained directly from the anode and the light obtained after reflection at the electrode interfere with each other. The thickness of the electron transporting layer is suitably selected in the range of several nm to several µm so that the interference is effectively utilized. When the thickness is great, it is preferable that the mobility of electrons is at least 10⁻⁵ cm²/Vs or greater under the application of an electric field of 10⁴ to 10⁶ V/cm so that the increase in the voltage is prevented.

As the material used for the electron injecting layer, metal complexes of 8-hydroxyquinoline and derivatives thereof and oxadiazole derivatives are preferable. Examples of the metal complexes of 8-hydroxyquinoline and the derivative thereof include metal chelated oxinoid compounds including chelate compounds of oxines (in general, 8-quinolinol or 8-hydroxyquinoline). For example, tris(8-quinolinol)aluminum can be used as the electron injecting material.

Examples of the oxadiazole derivative include electron transfer compounds represented by the following general formulae: 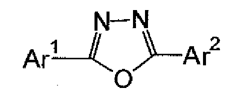 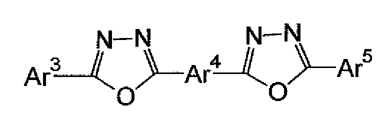 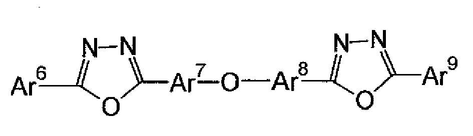 In the above formulae, Ar¹, Ar², Ar³, Ar⁵, Ar⁶ and Ar⁹ each represent a substituted or unsubstituted aryl group and may represent the same group or different groups. Ar⁴, Ar⁷ and Ar⁸ each represent a substituted or unsubstituted arylene group and may represent the same group or different groups.

Examples of the aryl group include phenyl group, biphenyl group, anthranyl group, perylenyl group and pyrenyl group. Examples of the arylene group include phenylene group, naphthylene group, biphenylene group, anthranylene group, perylenylene group and pyrenylene group. Examples of the substituent include alkyl groups having 1 to 10 carbon atoms, alkoxyl groups having 1 to 10 carbon atoms and cyano group. As the electron transfer compound, compounds which can form thin firms are preferable.

Specific examples of the electron transfer compound include the following compounds:

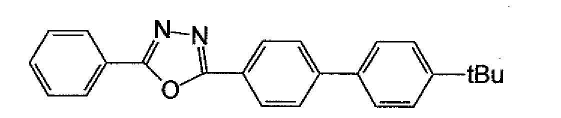

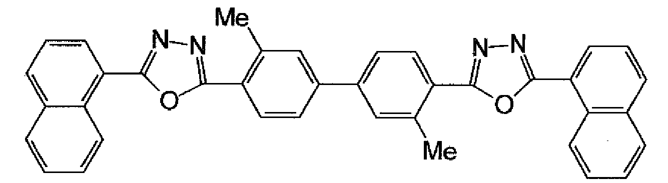

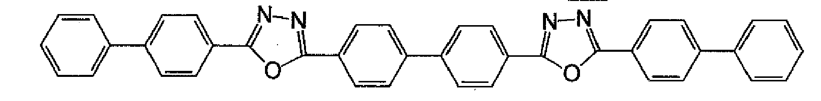

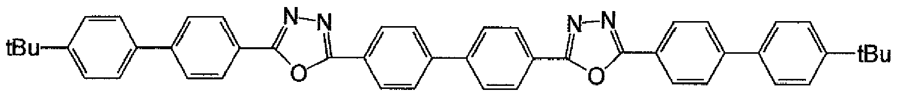

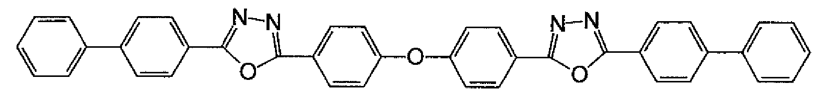

As the material which can be used for the electron injecting layer and the electron transporting layer, compounds represented by the following general formulae (E) to (J) can be used.

Heterocyclic derivatives having nitrogen atom represented by any one of general formulae (E) and (F):

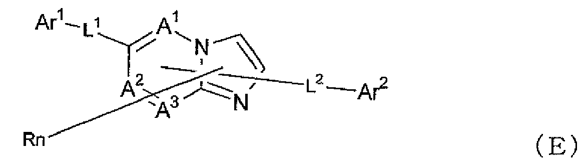

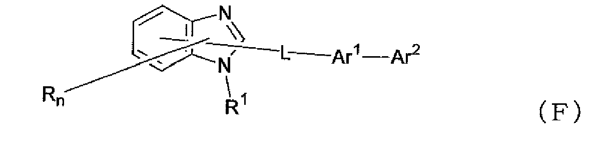

In general formulae (E) and (F), A¹ to A³ each independently represent nitrogen atom or carbon atom.

Ar¹ represents a substituted or unsubstituted aryl group having 6 to 60 ring carbon atoms or a substituted or unsubstituted heteroaryl group having 3 to 60 ring carbon atoms; Ar² represents hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms, a substituted or unsubstituted alkoxyl group having 1 to 20 carbon atoms or a divalent group derived from any of the above groups; and either one of Ar¹ and Ar² represents a substituted or unsubstituted condensed cyclic group having 10 to 60 ring carbon atoms or a substituted or unsubstituted monohetero condensed cyclic group having 3 to 60 ring carbon atoms.

L¹, L² and L each independently represent the single bond, a substituted or unsubstituted arylene group having 6 to 60 ring carbon atoms, a substituted or unsubstituted heteroarylene group having 3 to 60 ring carbon atoms or a substituted or unsubstituted fluorenylene group.

R represents hydrogen atom, a substituted or unsubstituted aryl group having 6 to 60 ring carbon atoms, a substituted or unsubstituted heteroaryl group having 3 to 60 ring carbon atoms, a substituted or unsubstituted alkyl group having 1 to 20 carbon atoms or a substituted or unsubstituted alkoxyl group having 1 to 20 carbon atoms; n represents an integer of 0 to 5; and, when n represents an integer of 2 or greater, the atoms and the groups represented by a plurality of R may be the same with or different from each other, and a plurality of groups represented by R which are adjacent to each other may be bonded to each other to form an aliphatic ring of the carbon ring type or an aromatic ring of the carbon ring type.

Heterocyclic compounds having nitrogen atom represented by the following general formula (G):

HAr-L-Ar¹-Ar² (G)

In general formula (G), HAr represents a heterocyclic group having 3 to 40 carbon atoms and nitrogen atom which may have substituents, L represents the single bond or an arylene group having 6 to 60 carbon atoms which may have substituents, a heteroarylene group having 3 to 60 carbon atoms which may have substituents or a fluorenylene group which may have substituents, Ar¹ represents a divalent aromatic hydrocarbon group having 6 to 60 carbon atoms which may have substituents, and Ar² represents an aryl group having 6 to 60 carbon atoms which may have substituents or a heteroaryl group having 3 to 60 carbon atoms which may have substituents.

Silacyclopentadiene derivatives represented by the following general formula (H):

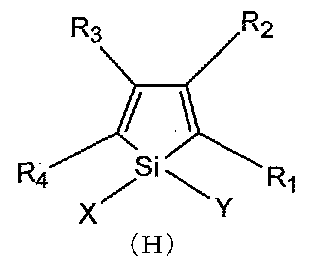

In general formula (H), X and Y each independently represent a saturated or unsaturated hydrocarbon group having 1 to 6 carbon atoms, an alkoxyl group, an alkenyloxyl group, an alkynyloxyl group, hydroxyl group, a substituted or unsubstituted aryl group, a substituted or unsubstituted heterocyclic group or a saturated or unsaturated cyclic group formed by bonding of the above groups represented by X and Y; and R₁ to R₄ each independently represent hydrogen atom, a halogen atom, a substituted or unsubstituted alkyl group having 1 to 6 carbon atoms, an alkoxyl group, an aryloxyl group, a perfluoroalkyl group, a perfluoroalkoxyl group, an amino group, an alkylcarbonyl group, an arylcarbonyl group, an alkoxycarbonyl group, an aryloxycarbonyl group, an azo group, an alkylcarbonyloxyl group, an arylcarbonyloxyl group, an alkoxycarbonyloxyl group, an aryloxycarbonyloxyl group, sulfinyl group, sulfonyl group, sulfanyl group, silyl group, carbamoyl group, an aryl group, a heterocyclic group, an alkenyl group, an alkynyl group, nitro group, formyl group, nitroso group, formyloxyl group, isocyano group, cyanate group, isocyanate group, thiocyanate group, isothiocyanate group, a cyano group or, when the groups are adjacent to each other, a structure formed by condensation of substituted or unsubstituted rings.

Borane derivatives represented by the following general formula (I):

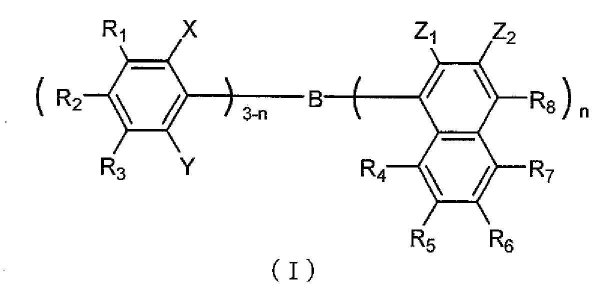

In general formula (I), R₁ to R₈ and Z₂ each independently represent hydrogen atom, a saturated or unsaturated hydrocarbon group, an aromatic hydrocarbon group, a heterocyclic group, a substituted amino group, a substituted boryl group, an alkoxyl group or an aryloxyl group; X, Y and Z₁ each independently represent a saturated or unsaturated hydrocarbon group, an aromatic hydrocarbon group, a heterocyclic group, a substituted amino group, an alkoxyl group or an aryloxyl group, and substituents to the groups represented by Z₁ and Z₂ may be bonded to each other to form a condensed ring; n represents an integer of 1 to 3 and, when n represents an integer of 2 or greater, a plurality of Z₁ may represent different groups; and the case where n represents 1, X, Y and R₂ each represent methyl group and R₈ represents hydrogen atom or a substituted boryl group and the case where n represents 3 and Z₁ represents methyl group are excluded.

Compounds represented by general formula (J):

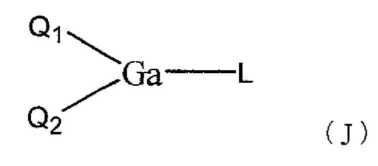

In general formula (J), Q₁ and Q₂ each independently represent a ligand represented by the following general formula (K): 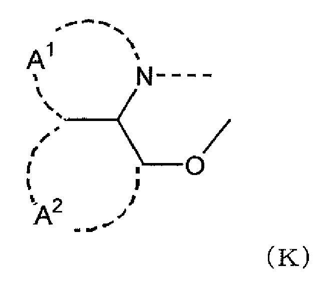 (rings A¹ and A² each representing six-membered aryl cyclic structure which may have substituents and are condensed with each other), and L represents a halogen atom, a substituted or unsubstituted alkyl group, a substituted or unsubstituted cycloalkyl group, a substituted or unsubstituted aryl group, a substituted or unsubstituted heterocyclic group or a ligand represented by -OR¹ (R¹ representing hydrogen atom, a substituted or unsubstituted alkyl group, a substituted or unsubstituted cycloalkyl group, a substituted or unsubstituted aryl group or a substituted or unsubstituted heterocyclic group) or -O-Ga-Q³(Q⁴) (Q³ and Q⁴ being as defined for Q¹ and Q²).

The above metal complex compound strongly exhibits the property as the n-type semiconductor and a great ability of electron injection, and the energy of formation of the complex compound is small. Therefore, the bonding between the metal and the ligand in the formed metal complex compound is strong, and the quantum efficiency of fluorescence as the light emitting material is great.

Examples of the substituent to rings represented by A¹ and A² forming the ligand represented by general formula (K) include halogen atoms such as chlorine atom, bromine atom, iodine atom and fluorine atom; substituted and unsubstituted alkyl groups such as methyl group, ethyl group, propyl group, butyl group, sec-butyl group, tert-butyl group, pentyl group, hexyl group, heptyl group, octyl group, stearyl group and trichloromethyl group; substituted and unsubstituted aryl groups such as phenyl group, naphthyl group, 3-methylphenyl group, 3-methoxyphenyl group, 3-fluorophenyl group, 3-trichloromethylphenyl group, 3-trifluoromethylphenyl group and 3-nitrophenyl group; substituted and unsubstituted alkoxyl groups such as methoxyl group, n-butoxyl group, tert-butoxyl group, trichloromethoxyl group, trifluoroethoxyl group, pentafluoropropoxyl group, 2,2,3,3- tetrafluoropropoxyl group, 1,1,1,3,3,3-hexafluoro-2-propoxyl group and 6-(perfluoroethyl)hexyloxyl group; substituted and unsubstituted aryloxyl groups such as phenoxyl group, p-nitrophenoxyl group, p-tert-butyl- phenoxyl group, 3-fluorophenoxyl group, pentafluorophenoxyl group and 3-trifluoromethylphenoxyl group; substituted and unsubstituted alkylthio groups such as methylthio group, ethylthio group, tert-butylthio group, hexylthio group, octylthio group and trifluoromethylthio group; substituted and unsubstituted arylthio groups such as phenylthio group, p-nitrophenylthio group, p-tert-butylphenylthio group, 3-fluorophenylthio group, pentafluorophenylthio group and 3-trifluoromethylphenylthio group; cyano group; nitro group; amino group; mono- and disubstituted amino groups such as methylamino group, diethylamino group, ethylamino group, dipropylamino group, dibutyl- amiono group and diphenylamino group; acylamino groups such as bis(acetoxymethyl)amino group, bis(acetoxyethyl)amino group, bis(acetoxypropyl)amino group and bis(acetoxybutyl)amino group; hydroxyl group; siloxyl group; acyl group; carbamoyl groups such as methylcarbamoyl group, dimethylcarbamoyl group, ethylcarbamoyl group, diethylcarbamoyl group, propylcarbamoyl group, butylcarbamoyl group and phenylcarbamoyl group; carboxylic acid group; sulfonic acid group; imide group; cycloalkyl groups such as cyclopentane group and cyclohexyl group; aryl groups such as phenyl group, naphthyl group, biphenyl group, anthranyl group, phenanthryl group, fluorenyl group and pyrenyl group; and heterocyclic groups such as pyridinyl group, pyrazinyl group, pyrimidinyl group, pyridazinyl group, triazinyl group, indolinyl group, quinolinyl group, acridinyl group, pyrrolidinyl group, dioxanyl group, piperidinyl group, morpholidinyl group, piperazinyl group, triatinyl group, carbazolyl group, furanyl group, thiophenyl group, oxazolyl group, oxadiazolyl group, benzoxazolyl group, thiazolyl group, thiadiazolyl group, benzothiazolyl group, triazolyl group, imidazolyl group, benzimidazolyl group and planyl group. The above substituents may be bonded to each other to form a six-membered aryl group or heterocyclic group.

A device comprising a reducing dopant in the interfacial region between a region transporting electrons or the cathode and the organic layer is preferable as an embodiment of the organic EL device of the present invention. The reducing dopant is defined as a substance which can reduce a compound having the electron transporting property. Various compounds can be used as the reducing dopant as long as the compounds have the specific reductive property. For example, at least one substance selected from the group consisting of alkali metals, alkaline earth metals, rare earth metals, oxides of alkali metals, halides of alkali metals, oxides of alkaline earth metals, halides of alkaline earth metals, oxides of rare earth metals, halides of rare earth metals, organic complexes of alkali metals, organic complexes of alkaline earth metals and organic complexes of rare earth metals can be advantageously used.

Preferable examples of the reducing dopant include substances having a work function of 2-9 eV or smaller, specific examples of which include at least one alkali metal selected from the group consisting of Na (the work function: 2.36 eV), K (the work function: 2.28 eV), Rb (the work function: 2.16 eV) and Cs (the work function: 1.95 eV) and at least one alkaline earth metal selected from the group consisting of Ca (the work function: 2.9 eV), Sr (the work function: 2.0 to 2.5 eV) and Ba (the work function: 2.52 eV). Among the above substances, at least one alkali metal selected from the group consisting of K, Rb and Cs is more preferable, Rb and Cs are still more preferable, and Cs is most preferable as the reducing dopant. These alkali metals have great reducing ability, and the luminance of the emitted light and the life of the organic EL device can be increased by addition of a relatively small amount of the alkali metal into the electron injecting zone. As the reducing dopant having a work function of 2.9 eV or smaller, combinations of two or more alkali metals are also preferable. Combinations having Cs such as the combinations of Cs and Na, Cs and K, Cs and Rb and Cs, Na and K are more preferable. The reducing ability can be efficiently exhibited by the combination having Cs. The luminance of emitted light and the life of the organic EL device can be increased by adding the combination having Cs into the electron injecting zone.

The organic EL device of the present invention may further comprise an electron injecting layer which is constituted with an insulating material or a semiconductor and disposed between the cathode and the organic layer. By the electron injecting layer, leak of electric current can be effectively prevented, and the electron injecting property can be improved. As the insulating material, at least one metal compound selected from the group consisting of alkali metal chalcogenides, alkaline earth metal chalcogenides, halides of alkali metals and halides of alkaline earth metals is preferable. It is preferable that the electron injecting layer is constituted with the above substance such as the alkali metal chalcogenide since the electron injecting property can be further improved. Preferable examples of the alkali metal chalcogenide include Li₂O, LiO, Na₂S, Na₂Se and NaO. Preferable examples of the alkaline earth metal chalcogenide include CaO, BaO, SrO, BeO, BaS and CaSe. Preferable examples of the halide of an alkali metal include LiF, NaF, KF, LiCl, KCl and NaCl. Preferable examples of the halide of an alkaline earth metal include fluorides such as CaF₂, BaF₂, SrF₂, MgF₂ and BeF₂ and halides other than the fluorides.

Examples of the semiconductor constituting the electron transporting layer include oxides, nitrides and oxide nitrides of at least one metal selected from Ba, Ca, Sr, Yb, Al, Ga, In, Li, Na, Cd, Mg, Si, Ta, Sb and Zn used singly or in combination of two or more. It is preferable that the inorganic compound constituting the electron transporting layer forms a fine crystalline or amorphous insulating thin film. When the electron injecting layer is constituted with the insulating thin film described above, a more uniform thin film can be formed, and defects of pixels such as dark spots can be decreased. Examples of the inorganic compound include alkali metal chalcogenides, alkaline earth metal chalcogenides, halides of alkali metals and halides of alkaline earth metals which are described above.

For the cathode, a material such as a metal, an alloy, a conductive compound or a mixture of these materials which has a small work function (4 eV or smaller) is used as the electrode material. Examples of the electrode material include sodium, sodium-potassium alloys, magnesium, lithium, magnesium-silver alloys, aluminum/aluminum oxide, Al/Li₂O, Al/LiO₂, Al/LiF, aluminum-lithium alloys, indium and rare earth metals.

The cathode can be prepared by forming a thin film of the electrode material described above in accordance with a process such as the vapor deposition process and the sputtering process.

When the light emitted from the light emitting layer is obtained through the cathode, it is preferable that the cathode has a transmittance of the emitted light greater than 10 %. It is also preferable that the sheet resistivity of the cathode is several hundred Ω/□ or smaller. The thickness of the cathode is, in general, selected in the range of 10 nm to 1 µm and preferably in the range of 50 to 200 nm.

Defects in pixels tend to be formed in organic EL device due to leak and short circuit since an electric field is applied to ultra-thin films. To prevent the formation of the defects, a layer of a thin film having an insulating property may be inserted between the pair of electrodes.

Examples of the material used for the insulating layer include aluminum oxide, lithium fluoride, lithium oxide, cesium fluoride, cesium oxide, magnesium oxide, magnesium fluoride, calcium oxide, calcium fluoride, aluminum nitride, titanium oxide, silicon oxide, germanium oxide, silicon nitride, boron nitride, molybdenum oxide, ruthenium oxide and vanadium oxide. Mixtures and laminates of the above compounds can also be used.

To prepare the organic EL device of the present invention, for example, the anode, the light emitting layer and, where necessary, the hole injecting layer and the electron injecting layer are formed in accordance with the above process using the above materials, and the cathode is formed in the last step. The organic EL device may be prepared by forming the above layers in the order reverse to that described above, i.e., the cathode being formed in the first step and the anode in the last step.

An embodiment of the process for preparing an organic EL device having a construction in which an anode, a hole injecting layer, a light emitting layer, an electron injecting layer and a cathode are disposed successively on a substrate transmitting light will be described in the following.

On a suitable substrate which transmits light, a thin film made of a material for the anode is formed in accordance with the vapor deposition process or the sputtering process so that the thickness of the formed thin film is 1 µm or smaller and preferably in the range of 10 to 200 nm. The formed thin film is used as the anode. Then, a hole injecting layer is formed on the anode. The hole injecting layer can be formed in accordance with the vacuum vapor deposition process, the spin coating process, the casting process or the LB process, as described above. The vacuum vapor deposition process is preferable since a uniform film can be easily obtained and the possibility of formation of pin holes is small. When the hole injecting layer is formed in accordance with the vacuum vapor deposition process, in general, it is preferable that the conditions are suitably selected in the following ranges: the temperature of the source of the deposition: 50 to 450°C; the vacuum: 10⁻⁷ to 10⁻³ Torr; the rate of deposition: 0.01 to 50 nm/second; the temperature of the substrate: -50 to 300°C and the thickness of the film: 5 nm to 5 µm; although the conditions of the vacuum vapor deposition are different depending on the used compound (the material for the hole injecting layer) and the crystal structure and the recombination structure of the hole injecting layer to be formed.

Then, the light emitting layer is formed on the hole injecting layer formed above. The light emitting layer can be obtained, using the light emitting material of the present invention, by forming a thin film of the light emitting material in accordance with the vacuum vapor deposition process, the sputtering process, the spin coating process or the casting process. The vacuum vapor deposition process is preferable since a uniform film can be easily obtained and the possibility of formation of pin holes is small. When the light emitting layer is formed in accordance with the vacuum vapor deposition process, in general, the conditions of the vacuum vapor deposition process can be selected in the same ranges as those described for the vacuum vapor deposition to form the hole injecting layer although the conditions are different depending on the used compound. It is preferable that the thickness is in the range of 10 to 40 nm.

The electron injecting layer is formed on the light emitting layer formed above. Similarly to the hole injecting layer and the light emitting layer, it is preferable that the electron injecting layer is formed in accordance with the vacuum vapor deposition process since a uniform film must be obtained. The conditions of the vacuum vapor deposition can be selected in the same ranges as those described for the vacuum vapor deposition to form the hole injecting layer and the light emitting layer.

The cathode is formed on the electron injecting layer formed above in the last step, and the organic EL device can be obtained. The cathode is made of a metal and can be formed in accordance with the vacuum vapor deposition process or the sputtering process. It is preferable that the vacuum vapor deposition process is used in order to prevent formation of damages on the lower organic layers during the formation of the film.

In the above preparation of the organic EL device, it is preferable that the above layers from the anode to the cathode are formed successively while the preparation system is kept in a vacuum after being evacuated.

The process for forming the layers in the organic EL device of the present invention is not particularly limited. A conventional process such as the vacuum vapor deposition process and the spin coating process can be used. The organic thin film layer which is used in the organic EL device of the present invention and comprises the compound represented by general formula (1) described above can be formed in accordance with a conventional process such as the vacuum vapor deposition process and the molecular beam epitaxy process (the MBE process) or, using a solution prepared by dissolving the compounds into a solvent, in accordance with a coating process such as the dipping process, the spin coating process, the casting process, the bar coating process and the roll coating process.

The thickness of each layer in the organic thin film layer in the organic EL device of the present invention is not particularly limited. A thickness in the range of several nanometers to 1 µm is preferable so that defects such as pin holes are decreased and the efficiency can be improved.

When a direct voltage is applied to the organic EL device, emission of light can be observed under application of a voltage of 5 to 40 V in the condition that the anode is connected to a positive electrode (+) and the cathode is connected to a negative electrode (-). When the connection is reversed, no electric current is observed and no light is emitted at all. When an alternating voltage is applied to the organic EL device, the uniform light emission is observed only in the condition that the polarity of the anode is positive and the polarity of the cathode is negative. When an alternating voltage is applied to the organic EL device, any type of wave shape can be used.

### EXAMPLES

The present invention will be described more specifically with reference to examples in the following. However, the present invention is not limited to the examples.

### Synthesis Example 1 (Synthesis of a compound (AN-1))

Under the atmosphere of argon, 10 g of 1-bromo-4-iodonaphthalene and 6 g of 4-bromophenylboronic acid which were synthesized in accordance with conventional processes were dissolved into 150 ml of toluene, and 45 ml of a 2M aqueous solution of sodium carbonate was added. Then, 1 g of tetrakistriphenylphosphinepalladium was added, and the resultant mixture was heated under the refluxing condition for 7 hours. After one night, the formed organic layer was extracted with toluene, washed with water and a saturated aqueous solution of sodium chloride. The organic layer was dried with anhydrous sodium sulfate, and the solvent was removed by distillation. The residue was purified in accordance with the silica gel column chromatography (the solvent of development: toluene/hexane), and 6.3 g of 1-bromo-4-(4-bromophenyl)-naphthalene was obtained (the yield: 58%).

The obtained 1-bromo-4-(4-bromophenyl)naphthalene in an amount of 6 g was mixed with 10 g of 10-phenylathracene-9-boronic acid synthesized in accordance with a conventional process and 150 ml of DME. Then, 1.1 g of tetrakistriphenylphosphinepalladium and 50 ml of a 2M aqueous solution of sodium carbonate were added, and the atmosphere was purged with argon. After the resultant mixture was heated under the refluxing condition for 7.5 hours, the mixture was cooled by leaving standing, and formed crystals were separated by filtration. The separated crystals were washed with water, methanol and then heated toluene, and 8.3 g of the object compound (AN-1) was obtained as a light yellow solid substance (the yield: 70%). When the obtained compound was examined in accordance with the field desorption mass spectroscopy (FDMS), it was found that m/z=708, which agreed with C₅₆H₃₆=708, and the above compound was identified to be Compound AN-1.

### Synthesis Example 2 (Synthesis of a compound (AN-3))

Under the atmosphere of argon, 25 ml of dehydrated tetrahydrofuran (THF) was added to 1.3 g of Mg for the Grignard reaction. Separately, 10 g of p-dibromobenzene was dissolved into 50 ml of dehydrated THF, and the obtained solution was mixed with the fluid containing Mg. After the reaction started, the reaction mixture was heated under the refluxing condition for 1 hour, and a THF solution of 4-bromophenylmagnesium bromide was prepared.

Under the atmosphere of argon, 12.5 g of 6-bromo-2-naphthyl trifluoromethanesulfonate, 0.85 g of PdCl₂(dppp) and 3 g of LiBr were dispersed into 60 ml of dehydrated THF, and the resultant dispersion was cooled with ice. To the cooled dispersion, the THF solution of 4-bromophenylmagnesium bromide prepared above was added, and the resultant mixture was stirred at the room temperature for 1 hour. After the mixture was heated under the refluxing condition for 5 hours, the mixture was cooled by leaving standing. The obtained mixture was adjusted at the acidic condition with a 10% dilute hydrochloric acid. The organic layer was extracted with toluene and washed with water and a saturated aqueous solution of sodium chloride. The organic layer was dried with anhydrous sodium sulfate, and the solvent was removed by distillation. The residue was purified in accordance with the silica gel column chromatography (the solvent of development: toluene/hexane), and 7.5 g of 2-bromo-6-(4-bromophenyl)naphthalene was obtained as a white solid substance (the yield: 58%).

In accordance with the same procedures as those conducted in Synthesis Example 1 except that 2-bromo-6-(4-bromophenyl)naphthalene was used in place of 1-bromo-4-(4-bromophenyl)naphthalene, the object compound (AN-3) was obtained as a light yellow solid substance (the yield: 57%). When the obtained compound was examined in accordance with FDMS, it was found that m/z=708, which agreed with C₅₆H₃₆=708, and the above compound was identified to be AN-3.

### Synthesis Example 3 (Synthesis of a compound (AN-6))

In accordance with the same procedures as those conducted in Synthesis Example 1 except that 1,4-dibromonaphthalene was used in place of 1-bromo-4-(4-bromophenyl)naphthalene and 4-(9-phenyl-anthracen-10-yl)phenylboronic acid was used in place of 10-phenyl-anthracene-9-boronic acid, the object compound (AN-6) was obtained as a light yellow solid substance (the yield: 72%). When the obtained compound was examined in accordance with FDMS, it was found that m/z=784, which agreed with C₆₂H₄₀=784, and the above compound was identified to be AN-6.

### Synthesis Example 4 (Synthesis of a compound (AN-12))

Under the atmosphere of argon, 8 g of 10-phenylanthracene-9-boronic acid and 7.7 g of 2,6-dibromonaphthalene which were synthesized in accordance with conventional processes were dissolved into 150 ml of DME, and 41 ml of a 2M aqueous solution of sodium carbonate was added. Then, 0.93 g of tetrakistriphenylphosphinepalladium was added, and the resultant mixture was heated under the refluxing condition for 8 hours. After one night, the formed precipitates were separated by filtration. The mother liquor of the filtration was treated by extraction with toluene, and the extract was washed with water and a saturated aqueous solution of sodium chloride. The organic layer was dried with anhydrous sodium sulfate, and the solvent was removed by distillation. The residue was washed with methanol and hexane, and 7.2 g of 2-bromo-6-(9-phenyl-anthracen-10-yl)naphthalene was obtained (the yield: 58%).

In accordance with the same procedures as those conducted in Synthesis Example 1 except that 2-bromo-6-(9-phenylanthracen-10-yl)-naphthalene was used in place of 1-bromo-4-(4-bromophenyl)naphthalene and 4-(9-(3-biphenyl)anthracen-10-yl)phenylboronic acid was used in place of 10-pheiaylanthracene-9-boronic acid, the object compound (AN-12) was obtained as a light yellow solid substance (the yield: 65%). When the obtained compound was examined in accordance with FDMS, it was found that m/z=784, which agreed with C₆₂H₄₀=784, and the above compound was identified to be AN-12.

### Synthesis Example 5 (Synthesis of a compound (AN-29))

In accordance with the same procedures as those conducted in Synthesis Example 4 except that 4-(9-(phenylanthracen-10-yl)phenylboronic acid was used in place of 10-phenylanthracene-9-boronic acid and 1,4-dibromonaphthalene was used in place of 2,6-dibromonaphthalane, 1-(4-bromonaphthalen-1-yl)-4-(9-phenylanthracen-10-yl)benzene was obtained as a light yellow solid substance (the yield: 62%).

Under the atmosphere of argon, 10 g of 2-bromo-6-(9-phenyl-anthracen-10-yl)naphthalene was dispersed into 150 ml of dehydrated THF. After the obtained dispersion was cooled at -63°C, 16 ml of a 1.6 M hexane solution of n-butyllithium was added. After the resultant mixture was stirred at -63°C for 30 minutes, the temperature was raised to 0°C and then lowered to -63°C. To the cooled reaction fluid, 14 g of triisopropyl boronate was added, and the obtained mixture was stirred at -63°C for 3 hours. After the reaction fluid was left standing for one night, the reaction fluid was adjusted at the acidic condition with a 10% dilute hydrochloric acid, and formed crystals were separated by filtration. The separated crystals were washed with water and toluene and dried, and 5.4 g of 6-(9-phenylanthracen-10-yl)naphthalene-2-boronic acid was obtained (the yield: 58%).

In accordance with the same procedures as those conducted in Synthesis Example 1 except that 1-(4-bromonaphthalen-1-yl)-4-(9-phenyl-anthracen-10-yl)benzene was used in place of 1-bromo-4-(4-bromophenyl)-naphthalene and 6-(9-phenylanthracen-10-yl)naphthalene-2-boronic acid was used in place of 10-phenylanthracene-9-boronic acid, and the object compound (AN-29) was obtained as a light yellow solid substance (the yield: 62%). When the obtained compound was examined in accordance with FDMS, it was found that m/z=834, which agreed with C₆₆H₄₂=834, and the above compound was identified to be AN-29.

### Example 1 (Preparation of an organic EL device)

A glass substrate (manufactured by GEOMATEC Company) of 25 mm×75 mm× 1.1 mm thickness having an ITO transparent electrode was cleaned by application of ultrasonic wave in isopropyl alcohol for 5 minutes and then by exposure to ozone generated by ultraviolet light for 30 minutes. The cleaned glass substrate having the transparent electrode was attached to a substrate holder of a vacuum vapor deposition apparatus. On the surface of the cleaned substrate at the side having the transparent electrode, a film of N,N'-bis(N,N'-diphenyl-4-aminophenyl)-N,N-diphenyl-4,4'-diamino-1,1'-biphenyl (referred to as a TPD232 film, hereinafter) having a thickness of 60 nm was formed in a manner such that the formed film covered the transparent electrode. The formed TPD232 film worked as the hole injecting layer. On the formed TPD232 film, a layer of N,N,N',N'-tetra(4-biphenyl)diaminobiphenylene (referred to as a TBDB layer, hereinafter) having a thickness of 20 nm was formed. The formed TBDB film worked as the hole transporting layer. On the formed TBDB film, AN-3 prepared above was vapor deposited to form a film having a thickness of 40 nm. At the same time, as the light emitting molecule, an amine compound BD1 shown below was vapor deposited in an amount such that the ratio of the amounts by weight of AN-3 to BD1 were 40:3. The formed film worked as the light emitting layer. On the formed film, a film of Alq shown in the following having a thickness of 10 nm was formed. This film worked as the electron injecting layer. On the film formed above, LiF (the source of LiF: manufactured by SAES GETTERS Company) as the reducing dopant and Alq were binary vapor deposited, and an Alq:Li film (the thickness: 10 nm) was formed as the electron injecting layer (the cathode). On the formed Alq:Li film, Al metal was vapor deposited to form the metal cathode, and an organic EL device was prepared.

The obtained organic EL device was examined by passing electric current. Blue light having a luminance of emitted light of 700 cd/m² was emitted under a voltage of 6.8 V and a current density of 10 mA/cm². The initial luminance was set at 1,000 cd/m², and the half life of the obtained organic EL device was measured. The result is shown in Table 1.

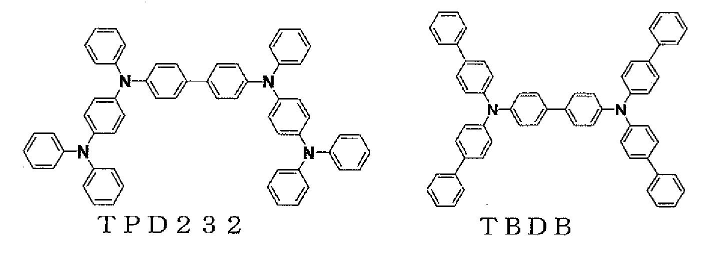

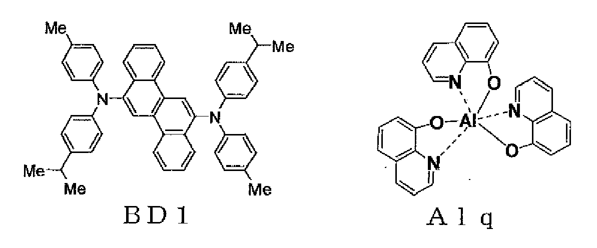

### Examples 2 to 5 (Preparation of organic EL devices)

Organic EL devices were prepared in accordance with the same procedures as those conducted in Example 1 except that the compounds shown in Table 1 were used as the material for the light emitting layer in place of AN-3. The half life of the obtained devices was measured in accordance with the same procedure as that conducted in Example 1. The results are shown in Table 1.

### Example 6

An organic EL device was prepared in accordance with the same procedures as those conducted in Example 1 except that an amine compound BD2 shown in the following was used as the material for the light emitting layer in place of the amine compound BD1. The half life of the obtained device was measured in accordance with the same procedure as that conducted in Example 1. The result is shown in Table 1.

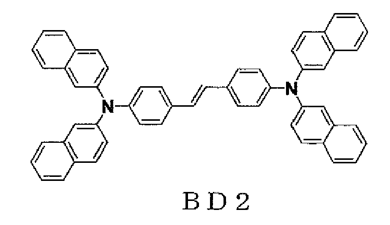

### Example 7

An organic EL device was prepared in accordance with the same procedures as those conducted in Example 1 except that an amine compound BD3 shown in the following was used as the material for the light emitting layer in place of the amine compound BD1. The half life of the obtained device was measured in accordance with the same procedure as that conducted in Example 1. The result is shown in Table 1.

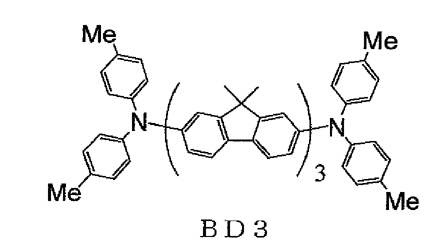

### Comparative Examples 1 to 8

Organic EL devices were prepared in accordance with the same procedures as those conducted in Example 1 except that the compounds shown in Table 1 were used as the materials for the light emitting layer in place of AN-3 and BD1. The half life of the obtained devices was measured in accordance with the same procedure as that conducted in Example 1. The results are shown in Table 1.

The structures of Compounds an-1 to an 6 described in Table 1 are shown in the following.

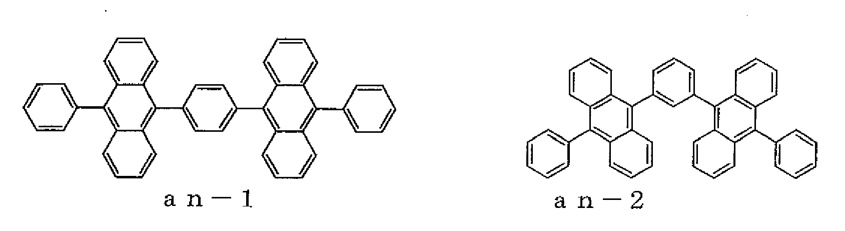

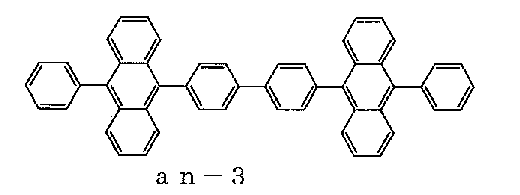

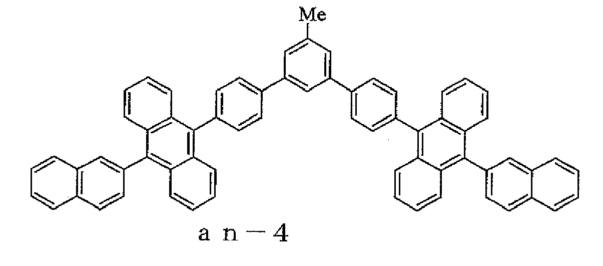

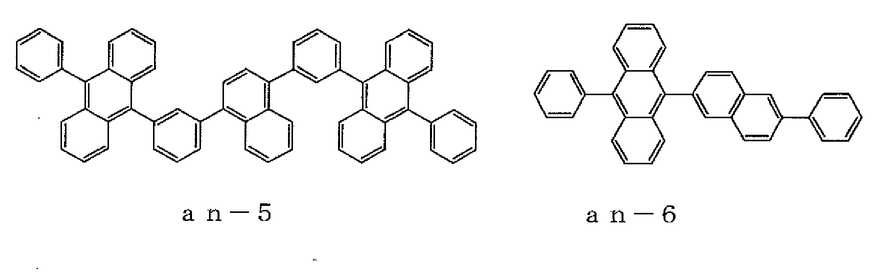

**Table 1**

| | Compound used in light emitting layer | Half life (hour) |
|---|---|---|
| Example 1 | AN-3BD-1 | 9,100 |
| Example 2 | AN-1/BD-1 | 8,300 |
| Example 3 | AN-6/BD-1 | 8,500 |
| Example 4 | AN-12/BD-1 | 8,700 |
| Example 5 | AN-29/BD-1 | 8,100 |
| Example 6 | AN-3/BD-2 | 7,200 |
| Example 7 | AN-3/BD-3 | 5,600 |
| Comparative Example 1 | an-1/BD-1 | 3,600 |
| Comparative Example 2 | an-2/BD-1 | 3,800 |
| Comparative Example 3 | an-3/BD-1 | 3,300 |
| Comparative Example 4 | an-4/BD-1 | 1,700 |
| Comparative Example 5 | an-5/BD-1 | 6,000 |
| Comparative Example 6 | an-6/BD-1 | 1,800 |
| Comparative Example 7 | an-5/BD-2 | 5,100 |
| Comparative Example 8 | an-5/BD-3 | 3,800 |

As shown in Table 1, the organic EL devices of Examples 1 to 7 in which the bisanthracene derivatives of the present invention represented by general formula (1) and having naphthylene group and p-phenylene group were used had longer lives than those of the organic EL devices of Comparative Examples 1 to 8 in which compounds having a structure not satisfying the above condition were used.

### INDUSTRIAL APPLICABILITY

As described specifically in the above, the organic EL device comprising the bisanthracene derivative of the present invention has a long life. Therefore, the organic EL device is very useful as the organic EL device which is expected to be used continuously for a long time.

## Claims

1. A bisanthracene derivative represented by following general formula (1): 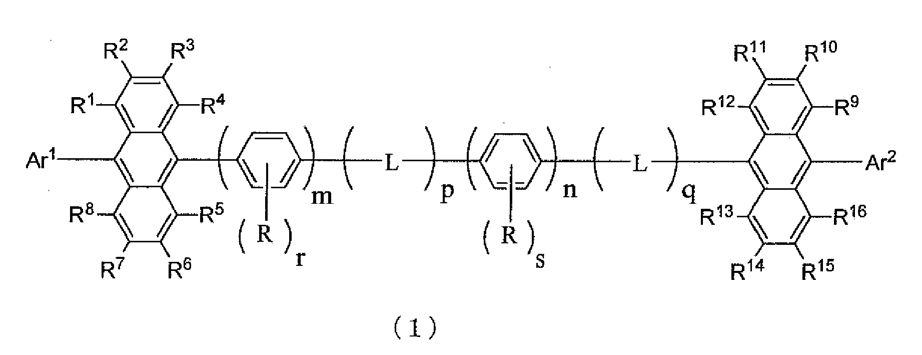 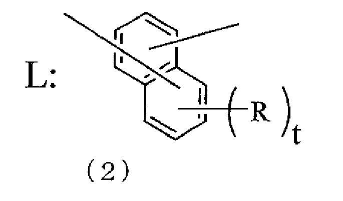 wherein
L represents a naphthylene group represented by above general formula (2);
Ar¹ and Ar² each independently represent a substituted or unsubstituted aromatic hydrocarbon group having 6 to 50 ring carbon atoms or a substituted or unsubstituted condensed aromatic hydrocarbon group having 10 to 50 ring carbon atoms;
R¹ to R¹⁶ each independently represent hydrogen atom, a substituted or unsubstituted aromatic hydrocarbon group having 6 to 50 ring carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 ring carbon atoms, a substituted or unsubstituted alkoxyl group having 1 to 50 carbon atoms, a substituted or unsubstituted aralkyl group having 6 to 50 carbon atoms, a substituted or unsubstituted aryloxyl group having 5 to 50 ring carbon atoms, a substituted or unsubstituted arylthio group having 5 to 50 ring carbon atoms, a substituted or unsubstituted alkoxycarbonyl group having 1 to 50 carbon atoms, a substituted or unsubstituted silyl group, carboxyl group, a halogen atom, cyano group, nitro group or hydroxyl group;
R represents a substituted or unsubstituted aromatic group having 6 to 50 ring carbon atoms, a substituted or unsubstituted aromatic heterocyclic group having 5 to 50 nuclear atoms, a substituted or unsubstituted alkyl group having 1 to 50 carbon atoms, a substituted or unsubstituted cycloalkyl group having 3 to 50 ring carbon atoms, a substituted or unsubstituted alkoxyl group having 1 to 50 carbon atoms, a substituted or unsubstituted aralkyl group having 5 to 50 ring carbon atoms and 6 to 50 carbon atoms, a substituted or unsubstituted aryloxyl group having 5 to 50 ring carbon atoms, a substituted or unsubstituted arylthio group having 5 to 50 ring carbon atoms, a substituted or unsubstituted alkoxycarbonyl group halving 1 to 50 carbon atoms, a substituted or unsubstituted silyl group, carboxyl group, a halogen atom, cyano group, nitro group or hydroxyl group;
m and n each represent an integer of 0 to 5, and m+n represents an integer of 1 to 5;
p and q each represent an integer of 0 to 5, and p+q represents an integer of 1 to 5;
r and s each represent an integer of 0 to 4;
t represents an integer of 0 to 6; and
when m, n, p, q, r, s and t represent integers of 1 or greater, atoms and groups represented by a plurality of L or R may be same with or different from each other.

2. A bisanthracene derivative according to Claim 1, wherein L in general formula (1) represents a naphthylene group represented by one of following general formulae (3) to (5): 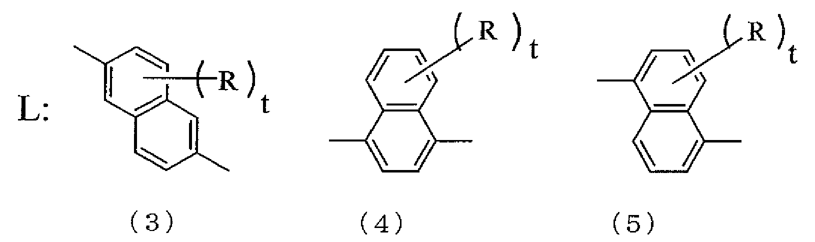 wherein R and t are as defined in Claim 1.

3. A bisanthracene derivative according to Claim 1, wherein m+n represents an integer of 1 to 3.

4. A bisanthracene derivative according to Claim 1, wherein p+q represents an integer of 1 to 3.

5. An organic electroluminescence device comprising a cathode, an anode and an organic thin film layer which comprises one layer or a plurality of layers comprising at least a light emitting layer and is disposed between the cathode and the anode, wherein the organic thin film layer comprises at least one compound selected from bisanthracene derivatives described in Claim 1 singly or as a component of a mixture.

6. An organic electroluminescence device according to Claim 5, wherein the light emitting layer comprises the bisanthracene derivative described in Claim 1 as a host material.

7. An organic electroluminescence device according to Claim 5, wherein the light emitting layer further comprises an arylamine compound.

8. An organic electroluminescence device according to Claim 5, wherein the light emitting layer further comprises a styrylamine compound.
